# EUROPÄISCHE PATENTSCHRIFT

(11) **EP 1 252 144 B1**
(45) Veröffentlichungstag und Bekanntmachung des Hinweises auf die Patenterteilung: **09.12.2009**
(21) Anmeldenummer: 01900400.1
(22) Anmeldetag: 09.01.2001
(51) Int. Cl.: C07D 211/88, A61K 31/45, A61P 37/02

(54) **SUBSTITUIERTE GLUTARIMIDE UND IHRE VERWENDUNG ALS INHIBITOREN DER IL-12 PRODUKTION**
SUBSTITUTED GLUTARIMIDES AND USE THEREOF IL-12 PRODUCTION INHIBITORS
GLUTARIMIDES SUBSTITUES ET LEUR UTILISATION EN TANT QU'INHIBITEURS DE LA PRODUCTION D'IL-12

(30) Priorität: 21.01.2000 DE 10002509
(43) Veröffentlichungstag der Anmeldung: 30.10.2002
(73) Patentinhaber: Grünenthal GmbH, 52078 Aachen (DE)
(72) Erfinder: GERMANN, Tieno, 52134 Herzogenrath (DE); FROSCH, Stefanie, 52078 Aachen (DE); WADE, Erik, 52068 Aachen (DE); BUSCHMANN, Helmut, 52066 Aachen (DE); ZIMMER, Oswald, 52146 Würselen (DE)
(86) Internationale Anmeldenummer: PCT/EP2001/000155
(87) Internationale Veröffentlichungsnummer: WO 2001/053261

(56) Entgegenhaltungen:
- EP-A- 0 989 121
- US-A- 5 114 937
- MOLLER D R ET AL: "Inhibition of IL-12 production by thalidomide" JOURNAL OF IMMUNOLOGY,US,THE WILLIAMS AND WILKINS CO. BALTIMORE, Bd. 159, Nr. 10, 1997, Seiten 5157-5161, XP002125907 ISSN: 0022-1767

## Beschreibung

Die Erfindung betrifft substituierte Glutarimide der allgemeinen Formel I 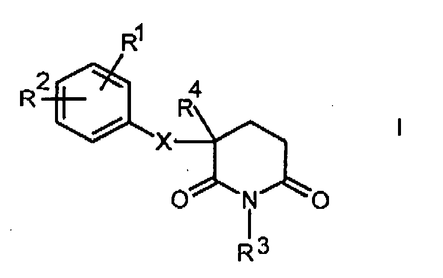 ihre Herstellung sowie ihre Verwendung in Arzneimitteln.

Autoimmunerkrankungen entstehen aufgrund einer Reaktivität des Immunsystems gegen körpereigene Strukturen. Dabei ist die normalerweise vorhandene Toleranz gegenüber körpereigenem Gewebe aufgehoben. In der Pathogenese der verschiedenen Autoimmunerkrankungen spielen neben Antikörpern insbesondere T-Lymphozyten und Monozyten/Makrophagen eine entscheidende Rolle. Aktivierte Monozyten/Makrophagen sezernieren eine Vielzahl verschiedener entzündungsfördernder Mediatoren, die direkt oder indirekt für die Zerstörung der von der Autoimmunerkrankung betroffenen Gewebe verantwortlich sind. Die Aktivierung von Monozyten/Makrophagen erfolgt entweder in der Interaktion mit T-Lymphozyten oder über bakterielle Produkte wie Lipopolysaccharid (LPS).

IL-12 ist ein heterodimeres Molekül, welches aus einer kovalent verbundenen p35 und p40 Kette besteht. Das Molekül wird von antigenpräsentierenden Zellen (Monozyten/Makrophagen, dendritische Zellen, B-Lymphozyten) gebildet. Die Bildung von IL-12 durch Monozyten/Makrophagen wird entweder durch verschiedene mikrobielle Produkte wie LPS, Lipopeptide, bakterielle DNA oder in der Interaktion mit aktivierten T-Lymphozyten ausgelöst (Trinchieri 1995. Ann.Rev.Immunol. 13: 251). IL-12 hat eine zentrale immunregulatorische Bedeutung und ist verantwortlich für die Entwicklung entzündungsfördernder TH1 Reaktivitäten. Beim Vorliegen einer TH1 Immunreaktion gegen Eigenantigene kommt es zum Auftreten schwerer Erkrankungen.

Die Bedeutung von entzündungsfördernden Zytokinen wie IL-12 für die Entwicklung und den Verlauf von Entzündungen und Autoimmunerkrankungen ist aufgrund zahlreicher tierexperimenteller und erster klinischer Untersuchungen klar dokumentiert. In verschiedenen Tiermodellen für Erkrankungen wie rheumatoide Arthritis, Multiple Sklerose, Diabetes mellitus sowie entzündliche Darm-, Haut- und Schleimhauterkrankungen zeigt sich die pathophysiologische Bedeutung von IL-12 (Trembleau et al. 1995. Immunol. Today 16: 383; Müller et al. 1995. J.Immunol. 155: 4661; Neurath et al. 1995. J.Exp.Med. 182: 1281; Segal et al. 1998. J.Exp.Med. 187: 537; Powrie et al. 1995. Immunity 3: 171; Rudolphi et al. 1996. Eur.J. Immunol. 26: 1156; Bregenholt et al. 1998. Eur.J. Immunol. 28: 379). Durch Applikation von IL-12 ließ sich die jeweilige Erkrankung auslösen bzw. nach Neutralisierung von endogenem IL-12 zeigte sich ein abgeschwächter Krankheitsverlauf bis hin zu einer Heilung der Tiere. Die Anwendung von Antikörpern gegen IL-12 am Menschen steht derzeit bevor.

Zusammenfassend läßt sich sagen, daß ein Überschuß an IL-12 die Pathophysiologie einer Vielzahl entzündlicher Erkrankungen bedingt. Ansätze zur Normalisierung des IL-12. Spiegels haben daher ein großes therapeutisches Potential.

Daneben ist IL-12 auch an der Regulation des Überlebens von Zellen beteiligt. Unkontrolliertes Zellwachstum wird u.a. durch Apoptose (programmierter Zelltod) reguliert. An T-Lymphozyten wurde gezeigt, daß IL-12 eine anti-apoptotische Wirkung besitzt und das Überleben von T-Zellen fördert (Clerici et al. 1994. Proc.Natl.Acad.Sci.USA 91: 11811; Estaquier et al. 1995. J.Exp.Med. 182: 1759). Eine lokale Überproduktion von IL-12 kann daher zum Überleben von Tumorzellen beitragen.

Inhibitoren der Bildung von IL-12 besitzen daher ein großes therapeutisches Potential.

Ein potenter Inhibitor der IL-12 Produktion ist der bekannte Wirkstoff Thalidomid (Journal of Immunology 159(10), 5157-5161 (1997)).

US 5114937 beschreibt Renin inhibierende Peptidabkömmlinge, deren Carboxamidgruppen durch deren Isostere ersetzt sind. Die Verbindungen eignen sich zur Behandlung von Renin assoziierte Hypertension, kongestivem Herzversagen, Hyperaldosteronismus, Glaukom und Erkrankungen, die durch die Retroviren HTLV-I, -II und -III hervorgerufen werden.

In DE 198 43 793.5 sind substituierte Benzamide mit immunmodulatorischen Eigenschaften vorgeschlagen worden, bei denen die ringhaltigen Strukturteile des Moleküls über eine Amidbindung miteinander verknüpft sind. Nachteil der Amidbindung ist die Anfälligkeit gegenüber Hydrolyse mit einhergehendem Wirkverlust für die Verbindung.

Die der Erfindung zugrundeliegende Aufgabe bestand somit in der Entwicklung von neuen Immunmodulatoren, die sich für die Behandlung und/oder Prophylaxe von Erkrankungen, die durch die Bildung des entzündungsfördernden Zytokins IL-12 hervorgerufen werden, eignen und gleichzeitig eine verbesserte Hydrolysestabilität aufweisen.

Diese an die zu entwickelnden Substanzen gestellten Anforderungen werden von bestimmten substituierten Glutarimiden erfüllt.

Gegenstand der Erfindung sind dementsprechend substituierte Glutarimide der Formel I 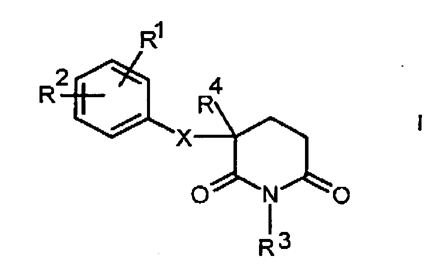 in der
- X: eine Gruppe der Formel (CH₂)ₙ-(CR⁸R⁹)ₚ-Z-(CR⁸R⁹)ₘ bedeutet,
- Z: für ein Schwefel- oder Sauerstoffatom, die SO- oder SO₂-Gruppe, den Rest NR⁸ (gegebenenfalls in Form des N-oxids) oder eine CR⁸R⁹-Gruppe,
- m und p: für 0 oder 1,

- n: für 0, 1, 2 oder 3 stehen, wobei m, n und p nicht gleichzeitig 0 sein können,
- R¹ und R²: gleich oder verschieden sind und für die Carboxylgruppe, eine Estergruppe der Formel COOR⁵ oder eine Acylgruppe der Formel COR⁵, in denen R⁵ jeweils eine Alkylgruppe (geradkettig oder verzweigt) mit 1 bis 6 C-Atomen (gegebenenfalls substituiert mit dem Rest GOOR⁵ und/oder einer Phenylgruppe), eine C₃- bis C₇-Cycloalkylgruppe oder einen Phenyl- oder Benzylrest bedeutet, oder eine Amidgruppe der Formel CONR⁶R⁷, in der R⁶ und R⁷ gleich oder verschieden sind und Wasserstoff, eine Alkylgruppe (geradkettig oder verzweigt) mit 1 bis 6 C-Atomen (gegebenenfalls substituiert mit dem Rest COOR⁵ und/oder einer Phenylgruppe), den Allylrest, den Phenylrest oder zusammengenommen mit dem N-Atom die Hydrazidgruppe, den Pyrrolidin-, Piperidin-, Hexamethylenimin-, Morpholin-, Thiomorpholin-, Piperazin- oder N-Methylpiperazinring darstellen, für Wasserstoff, Brom, Chlor, Fluor, eine Mono-, Di- oder Trifluormethyl-, Trityl-, Hydroxy-, Hydroxymethyl-, Trifluormethoxy-, Nitro-, Amino- (gegebenenfalls substituiert mit dem Rest CH(=O)oder COR⁵ oder einer Alkylsulfonylgruppe) oder Dimethylaminogruppe, einen Alkyl- oder Alkoxyrest (geradkettig oder verzweigt) mit 1 bis 6 C-Atomen, einen Amidinrest der Formel NH-CH(=NH) oder NH- C(=NH)R⁵, einen Phenylrest oder einen ankondensierten Benzolring(jeweils gegebenenfalls substituiert mit vorgenannten Atomen oder Gruppen, d.h. mit Wasserstoff, Brom, Chlor, Fluor, eine Mono-, Di- oder Trifluormethyl-, Trityl-, Hydroxy-, Hydroxymethyl-, Trifluormethoxy-, Nitro-, Amino- (gegebenenfalls substituiert mit dem Rest CH(=O) oder COR⁵ oder einer Alkylsulfonylgruppe) oder Dimethylaminogruppe, einen Alkyl- oder Alkoxyrest (geradkettig oder verzweigt) mit 1 bis 6 C-Atomen, einen Amidinrest der Formel NH-CH(=NH) oder NH- C(=NH)R⁵), mit der Einschränkung, daß für den Fall Z=CR⁸R⁹ R¹ und R² nicht gleichzeitig Wasserstoff und für den Fall Z = S und m = 0 nicht die Methoxygruppe darstellen können, stehen,
- R³: für Wasserstoff, den Hydroxyrest oder eine Gruppe der Formel CH₂-NR⁶R⁷ in der R⁶ und R⁷ wie oben definiert sind, steht,
- R⁴: für Wasserstoff, eine C₁ bis C₃-Alkylgruppe, ein Fluoratom, die Difluor- oder Trifluormethylgruppe steht,
- R⁸: für Wasserstoff, eine Alkylgruppe mit 1 bis 4 C-Atomen (geradkettig oder verzweigt), den Benzyl- oder Phenethylrest (gegebenenfalls substituiert mit vorgenannten Atomen oder Gruppen, d.h. mit Wasserstoff, Brom, Chlor, Fluor, eine Mono-, Di- oder Trifluormethyl-, Trityl-, Hydroxy-, Hydroxymethyl-, Trifluormethoxy-, Nitro-, Amino- (gegebenenfalls substituiert mit dem Rest CH(=O)oder COR⁵ oder einer Alkylsulfonylgruppe) oder Dimethylaminogruppe, einen Alkyl- oder Alkoxyrest (geradkettig oder verzweigt) mit 1 bis 6 C-Atomen, einen Amidinrest der Formel NH-CH(=NH) oder NH- C(=NH)R⁵) steht,
- und R⁹: dieselbe Bedeutung wie R⁸ hat, für die Estergruppe der Formel COOR⁵, den Phenylrest, die Hydroxygruppe oder einen Alkoxyrest (geradkettig oder verzweigt) mit 1 bis 4 C- Atomen, ein Fluor- oder Chloratom oder die Trifluormethylgruppe steht,
und deren Enantiomere, Enantiomerengemische, Racemate, Diastereomere oder Diastereomerengemische in Form ihrer Basen oder Salze physiologisch verträglicher Säuren.

Bevorzugte erfindungsgemäße Verbindungen sind solche, in denen X für die Gruppen CH₂-N und S-CH₂ steht, R¹ für die Carboxygruppe, eine Estergruppe der Formel CO0R⁵ wie oben definiert, eine Acylgruppe der Formel COR⁵ wie oben definiert oder eine Amidgruppe der Formel CONR⁶R⁷, in der R⁶ und R⁷ gleich oder verschieden sind und Wasserstoff, eine Alkylgruppe (geradkettig oder verzweigt) mit 1 bis 6 C-Atomen (gegebenenfalls substituiert wie oben definiert),den Phenylrest oder die zusammengenommen mit dem N-Atom die Hydrazidgruppe, den Pyrrolidin- oder Morpholidinring darstellen, steht, R² für Wasserstoff, die Nitro- oder Aminogruppe, R³ für Wasserstoff und R⁴ für Wasserstoff, Methyl oder Fluor stehen.

Besonders bevorzugt sind folgende substituierte Glutarimides:
2-[(3S)-(2,6-Dioxo-piperidin-3-ylamino)methyl]-benzoesäure
2-[(3R)-(2,6-Dioxo-piperidin-3-ylamino)methyl]-benzoesäure
2-[(3S)-(2,6-Dioxo-piperidin-3-ylamino)methyl]-N,N-diethylbenzamid
(3S)-[2-(Morpholin-4-carbonyl)benzylamino]-piperidin 2,6-dion
{2-[(3S)-(2,6-Dioxo-piperidin-3-ylamino)methyl] benzoylamino}-essigsäuremethylester
2-[(3S)-(2,6-Dioxo-piperidin-3-ylamino)methyl]-benzamid
2-[(3S)-(2,6-Dioxo-piperidin-3-ylamino)methyl]-N-ethylbenzamid
(3S)-[2-(Pyrrolidin-1-carbonyl)benzylamino]-piperidin-2,6-dion
2-[(3S)-(2,6-Dioxo-piperidin-3-ylamino)methyl]-benzoesäurehydrazid
2-[(3S)-(2,6-Dioxo-piperidin-3-ylamino)methyl]-N-phenylbenzamid
2-[(3R)-(2,6-Dioxo-piperidin-3-ylamino)methyl]-N-phenylbenzamid
2-[(3R)-(2,6-Dioxo-piperidin-3-ylamino)methyl]-N,N-diethyl-benzamid
2-[(3R)-(2,6-Dioxo-piperidin-3-ylamino)methyl]-benzamid
2-[(3S)-(2,6-Dioxo-piperidin-3-ylamino)-methyl]-benzoesäuremethylester
2-[(3S)-(2,6-Dioxo-piperidin-3-ylamino)-methyl]-benzoesäurebenzylester
2-(2,6-Dioxo-piperidin-3-ylmethyl)sulfanyl)-benzoesäuremethylester
2-(2,6-Dioxo-piperidin-3-ylmethylsulfanyl)-6-nitro-benzoesäuremethylester

Ein weiterer Gegenstand der vorliegenden Erfindung sind Verfahren zur Herstellung der erfindungsgemäßen Verbindungen der allgemeinen Formel I.

Verbindungen der allgemeinen Formel I, in der R³ für Wasserstoff oder den Hydroxyrest steht, lassen sich dadurch erhalten, dass man Glutarsäurederivate der allgemeinen Formel II, 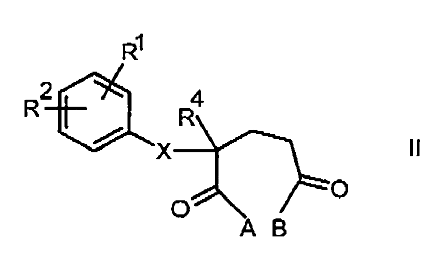 in der X, R¹, R² und R⁴ dieselbe Bedeutung wie oben haben, A für OH, B für NH₂ oder NHOH oder umgekehrt stehen, in Gegenwart aktivierender Reagenzien wie zum Beispiel Carbonyldiimidazol, cyclisiert. Bedeutet in der Verbindung der Formel I innerhalb X der Rest Z eine NH-Gruppe, so wird der Ringschluß vorzugsweise mit Verbindungen der Formel II durchgeführt, bei denen die NH-Funktion in geschützter Form, beispielsweise mit einer Benzyloxycarbonylgruppe, vorliegt. Diese wird anschließend, z.B. mit einer Lösung von Bromwasserstoff in Essigsäure, bei Temperaturen von 20 - 40°C abgespalten.

Stehen in der Formel II A und B für OH, so läßt sich, durch Erhitzen in Acetanhydrid, zunächst ein Ringschluß zum cyclischen Anhydrid erreichen, aus dem durch Erhitzen mit Harnstoff oder einer anderen Stickstoffquelle die Verbindung der Formel I mit R³ = H erhalten wird. Daraus lassen sich durch Umsetzung mit Paraformaldehyd oder einer wässrigen Formaldehydlösung und einem sekundären Amin der Formel HNR⁶R⁷, wobei R⁶ und R⁷ wie oben definiert sind, Verbindungen der allgemeinen Formel I mit R³ = CH₂ - NR⁶R⁷ herstellen.

Verbindungen der allgemeinen Formel I mit R³ - H lassen sich auch aus Lactamen der allgemeinen Formel III, 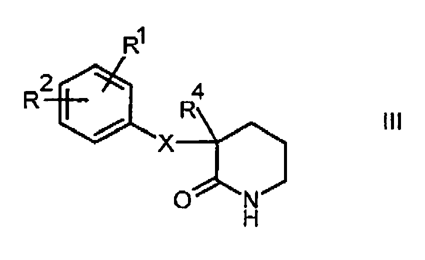 in der R¹, R², R⁴ und X dieselben Bedeutungen wie oben haben, herstellen, indem man die Verbindung III zum Imid oxidiert, vorzugsweise mit m-Chlor-perbenzoesäure oder Ruthenium (IV)-oxid/Natriumperiodat.

In Verbindungen der allgemeinen Formel I, in denen R¹ bis R³ und X dieselben Bedeutungen wie oben haben und R⁴ für Wasserstoff steht, läßt sich durch an sich bekannte Alkylierungs- oder Halogenierungsreaktionen dieser Wasserstoff gegen die übrigen definitionsgemäßen R⁴-Substituenten austauschen.

Ist für die Guppe X in den Verbindungen der Formel I m = 0 und steht Z für den Rest NR⁸, wobei R^{θ}, und n und p dieselben Bedeutungen wie oben haben, so können diese durch Alkylierung von α-Aminoglutarimiden der allgemeinen Formel IV, 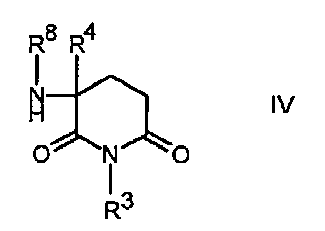 in der R³, R⁴ und R⁸ dieselben Bedeutungen wie oben haben, mit Verbindungen der allgemeinen Formel V, 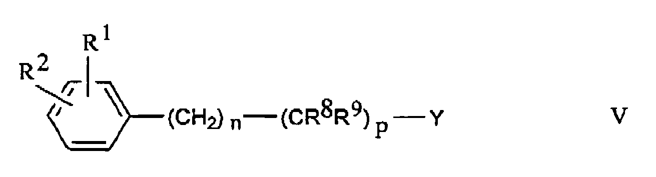 in der R¹, R², R⁸, R⁹, n und p dieselben Bedeutungen wie oben haben und Y für ein Chlor-, Brom- oder lodatom oder den Toluol-4-Sulfonatrest steht, erhalten werden.

Solche Verbindungen, in denen zudem p für 1 und R⁸ oder R⁹ für Wasserstoff stehen, können auch durch reduktive Aminierung aus Verbindungen der allgemeinen Formeln VI und IV, in denen R¹, R², R⁴, R⁸ und n dieselben Bedeutungen wie oben haben und R³ für Wasserstoff oder die Hydroxygruppe steht, erhalten werden.

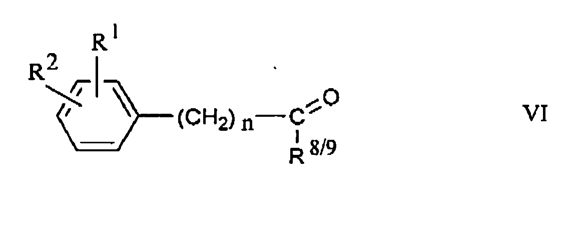

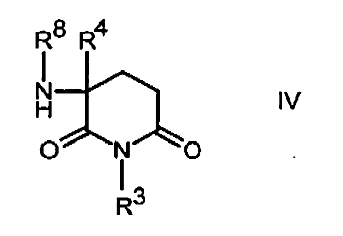

Als Reduktionsmittel dienen dabei vorzugsweise Natriumborhydrid, Natriumtriacetoxyborhydrid, Natriumcyanoborhydrid, der Boran-Pyridinkomplex oder katalytisch angeregter Wasserstoff.

Steht in der Gruppe X der Verbindung der Formel I m für 0 und Z für O, S oder NR⁸ und sind R⁸, n und p wie oben definiert, so sind diese Verbindungen auch durch Alkylierung einer Verbindung der allgemeinen Formel VII, 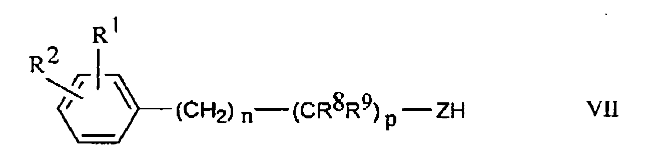 mit α-Bromglutarimiden der allgemeinen Formel VIII, 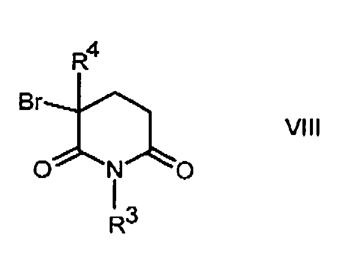 in der R³ und R⁴ wie oben definiert sind, erhältlich.

Verbindungen der allgemeinen Formel I mit R⁴ = Wasserstoff, bei denen in der Gruppe X n und p wie oben definiert sind, m für 1, Z für O, S oder NR⁸ und in der Gruppe CR⁸R⁹ mindestens einer der Reste R⁸ oder R⁹ für Wasserstoff stehen, können dadurch erhalten werden, daß eine Verbindung der allgemeinen Formel VII an ein 3-Methylenglutarimid der allgemeinen Formel IX addiert wird.

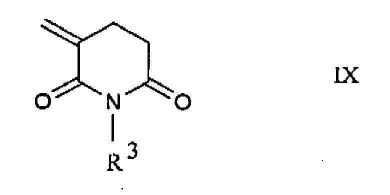

Die Umsetzung wird vorzugsweise in Lösungsmitteln wie Acetonitril oder Toluol unter Zusatz von tertiären Aminen wie beispielsweise Triethylamin oder Diisopropyl-ethylamin bei Temperaturen von 80 - 110°C durchgeführt.

Allgemein lassen sich aus Verbindungen der Formel I, in denen R¹ und/oder R² für eine Aminogruppe stehen, durch Reduktion von Verbindungen der Formel I mit R¹ und/oder R² = NO₂ erhalten. Die Reduktion erfolgt beispielsweise durch katalytisch angeregten Wasserstoff in säurehaitigen organischen Lösungsmitteln wie Essigsäureethylester, wobei bevorzugt Palladiumkatalysatoren eingesetzt werden. Alternativ kann die Reduktion mit Metallen wie Zinn oder Eisen in saurer Lösung durchgeführt werden.

Steht Z in der Gruppe X für SO oder SO₂, können solche Verbindungen der Formel I durch stufenweise Oxidation des entsprechenden Thioethers (Z = S) erhalten werden. Als Oxidationsmittel stehen Wasserstoffperoxid in essigsaurer Lösung, m-Chlorperbenzoesäure, tert-Butylhydroperoxid oder Oxone zur Verfügung, wobei letzteres bevorzugt zur Herstellung der Sulfone (Z = SO₂) dient. Die Oxidation zu Sulfoxiden (Z = SO) kann auch asymmetrisch gestaltet werden, etwa durch Einsatz des Sharpless-Systems bzw. des Davis-Reagenz oder über enzymatische Methoden.

Steht Z in der Gruppe X für den Rest NR⁸, so kann dieser in das entsprechende N-Oxid übergeführt werden, wobei bevorzugt Wasserstoffperoxid als Oxidationsmittel geeignet ist.

Die erfindungsgemäßen Verbindungen besitzen immunmodulatorische Aktivität, die sich in einer Inhibition der Produktion von IL-12 durch LPS-aktivierte Monozyten zeigt. Sie weisen außerdem im Vergleich zu bereits vorgeschlagenen Verbindungen eine verbesserte HydrolyseStabilität auf. Sie eignen sich zur Behandlung und/oder Prophylaxe von Entzündungs- sowie Autoimmunerkrankungen und auch von hämatologisch-onkologischen Erkrankungen.

Zu den Erkrankungen oben genannter Formenkreise zählen unter anderem Entzündungen der Haut (z.B. atopische Dermatitis, Psoriasis, Ekzeme), Entzündungen der Atemwege (z.B. Bronchitis, Pneumonie, Asthma bronchiale, ARDS (adult respiratory distress syndrome), Sarkoidose, Silikose/Fibrose), Entzündungen des Gastrointestinaltraktes (z.B. gastroduodenale Ulcera, Morbus Crohn, ulcerative Colitis), ferner Erkrankungen wie Hepatitis, Pankreatitis, Appendizitis, Peritonitis, Nephritis, Aphthosis, Konjunktivitis, Keratitis, Uveitis, Rhinitis.

Die Autoimmunerkrankungen umfassen z.B. Erkrankungen des arthritischen Formenkreises (z.B. rheumatoide Arthritis, HLA-B27 assoziierte Erkrankungen), Morbus Behcet ferner Multiple Sklerose, jugendlicher Diabetes oder Lupus erythematosus.

Weitere Indikationen sind Sepsis, bakterielle Meningitis, Kachexie, Transplantat-Abstoßungs-Reaktionen, Graft-versus-Host Reaktionen sowie das Reperfusions-Syndrom und Atherosklerose sowie Angiopathien (wie Maculadegeneration, diabetische Retinopathien).

Ferner gehören hämatologische Erkrankungen wie multiples Myelom und Leukämien sowie weitere onkologische Erkrankungen wie z. B. Glioblastom, Prostatacarcinom sowie Mammacarcinom zu den Krankheitsbildern, die durch eine Reduktion von IL-12 zu inhibieren sind.

Erfindungsgemäße Arzneimittel enthalten neben mindestens einer Verbindung der allgemeinen Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, rektal, ophthalmisch (intravitreal, intracameral), nasal, topisch (einschließlich buccal und sublingual), vaginal oder parenteral (einschließlich subkutan, intramuskulär, intravenös, intradermal, intratracheal und epidural) verabreicht werden soll.

Für die orale Applikation eignen sich Zubereitungen in Form von Tabletten, Kautabletten, Dragees, Kapseln, Granulaten, Tropfen, Säften oder Sirupen, für die parentale, topische und inhalative Applikation Lösungen, Suspensionen, leicht rekonstituierbare Trockenzubereitungen sowie Sprays. Kutane Applikationsformen sind Salben, Gele, Cremes und Pasten. Ophthalmische Applikationsiormen umfassen Tropfen, Salben und Gele. Erfindungsgemäße Verbindungen in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die an Patienten zu verabreichende Wirkstoffmenge variiert in Abhängigkeit vom Gewicht des Patienten, von der Applikationsart, der Indikation und dem Schweregrad der Erkrankung. Üblicherweise werden 1 bis 150 mg/kg wenigstens einer erfindungsgemäßen Verbindung der Formel I appliziert.

### Beispiele

Die folgenden Beispiele dienen zur näheren Erläuterung der vorliegenden Erfindung.

Als stationäre Phase für die chromatographischen Trennungen wurde Kieselgel 60 ( 0.040 - 0.063 mm ) der Firma E. Merck, Darmstadt, eingesetzt. Die Mischungsverhältnisse der Elutionsmittel sind stets in Volumen/Volumen angegeben.

Die Substanzen wurden über ihren Schmelzpunkt und/oder das ¹H-NMR-Spektrum charakterisiert. Die Aufnahme der Spektren erfolgte bei 300 MHz mit dem Gerät Gemini 300 der Firma Varian. Die chemischen Verschiebungen sind in ppm angegeben (δ-Skala ). Als interner Standard wurde Tetramethylsilan ( TMS ) verwendet.

### Beispiel 1

### 3-(2-Chlorbenzylamino)-piperidin-2,6-dion; Hydrochlorid

### Stufe 1:

### 3-Brom-piperidin-2,6-dion

10,2 g Glutarimid, suspendiert in 20 ml Chloroform, wurden mit 4,5 ml Brom versetzt und das Gemisch in einem geschlossenen Gefäß 90 Minuten bei einer Badtemperatur von 110°C gerührt. Nach dem Abkühlen wurde das Gefäß geöffnet und solange weitergerührt, bis kein Bromwasserstoff mehr entwich. Das Reaktionsgemisch wurde im Vakuum eingedampft, der Rückstand in Ethanol gelöst und erneut eingedampft. Dabei blieben 17,1 g (99 %) der Titelverbindung in Form nahezu weißer Kristalle zurück, die bei 76 - 83°C schmolzen.

### Stufe 2:

### 3-(2-Chlorbenzylamino)-piperidi.n-2,6-dion; Hydrochlorid

Eine Lösung von 0,39 g des Produkts aus Stufe 1 und 0,71 g 2-Chlorbenzylamin in 8 ml N,N-Dimethylformamid wurde bei 20°C 36 Stunden gerührt. Nach Eindampfen im Vakuum wurde der ölige Rückstand in 25 ml Methanol gelöst und die Lösung mit 1 g Amberlyst A-21 zwei Stunden gerührt. Man filtrierte ab, versetzte das Filtrat mit 2 g Kieselgel und engte zur Trockne ein. Die adsorbierte Substanz wurde auf eine Chromatographiesäule gegeben und das Produkt mit einem Gemisch Essigsäureethylester/Cyclohexan (1/2 → 1/1), das 1 % Triethylamin enthielt, eluiert. Der nach Eindampfen der Produktfraktionen verbleibende Rückstand wurde in 10 ml Methanol gelöst und die Lösung mit je 25 ml mit Chlorwasserstoff gesättigtem Diethylether bzw. Diethylether versetzt. Das ausgefallene Hydrochlorid wurde abgetrennt und aus Methanol/Diethylether umkristallisiert. Dabei fielen 0,24 g (41 % der Theorie) der Titelverbindung in Form von Kristallen an, die bei 217°C unter Zersetzung schmolzen.
¹H-NMR (DMSO-d₆):2,15 - 2,34 (1H, m); 2,40 - 2,56 (1H, m); 2, 60 - 2,80 (2H, m); 4,35 (1H, t, J - 13,5 Hz) ; 4,45 (2H, d, J = 13,8 Hz); 7,40 - 7,94 (4H, m).

### Beispiel 2

Unter Anwendung der in Beispiel 1, Stufe 2, beschriebenen Verfahrensweise und unter Einsatz entsprechender Benzylamine wurden analog erhalten:
2.1: 3-(2-Trifluormethyl benzylamino)-piperidin-2,6-dion; Hydrochlorid
   Schmelzpunkt: > 250°C (Zersetzung)
2.2: 3-(2,4-Dimethoxybenzylamino)-piperidin-2,6-dion; Hydrochlorid
   Schmelzpunkt: 214°C (Zersetzung)
2.3: 3-(2,6-Ditluorbenzylamino)-piperidin-2,6-dion; Hydrochlorid
   Schmelzpunkt: 208 - 215°C (Zersetzung)
2.4: 3-(2,5-Difluorbenzylamino)-piperidin-2,6-dion, Hydrochlorid
   Schmelzpunkt: 208°C (Zersetzung)
2.5: 3-(3,5-Difluorbenzylamino)-piperidin-2,6-dion; Hydrochlorid
   Schmelzpunkt: 230 - 236°C (Zersetzung)
2.6: 3-[(Naphth-1-ylmethyl)amino]-piperidin-2,6-dion; Hydrochlorid
   Schmelzpunkt: 188°C (Zersetzung)
2.7: 3-(2,3-Difluorbenzylamino)-piperidin-2,6-dion; Hydrochlorid
   Schmelzpunkt: 206 - 212°C (Zersetzung)
2.8: 3-(4-Dimethylaminobenzylamino)-piperidin-2,6-dion; Base
2.9: 3-(4-Nitrobenzylamino)-piperidin-2,6-dion: Hydrochlorid
2.10: 3-(3-Tritluormethylbenzylamino)-piperidin-2,6-dion; Hydrochlorid
2.11: 3-(3-Trifluormethoxybenzylamino)-piperidin-2,6-dion; Hydrochlorid
   Schmelzpunkt: 199 - 201°C
2.12: 3-[(Naphth-2-ylmethyl)amino]-piperidin-2,6-dion, Base
   Schmelzpunkt: 120 - 125°C (Zersetzung)
2.13: 3-((2-Chlor-4-fluorbenzylamino)-piperidin-2,6-dion; Hydrochlorid
   Schmelzpunkt: 241 - 242°C
2.14: 3-(3-Nitrobenzylamino)-piperidin-2,6-dion; Hydrochlorid
   Schmelzpunkt: ab 240°C unter Zersetzung
2.15: 3-(2-Chlor-6-methylbenzylamino)-piperidin-2,6-dion; Hydrochlorid
   Schmelzpunkt: 238 - 240°C
2.16: 3-(2-Methylbenzylamino)-piperidin-2,6-dion; Hydrochlorid
   Schmelzpunkt: 235 - 240°C
2.17: 3-(3,5-Dichlorbenzylamino)-piparidin-2,6-dion; Hydrochlorid
   Schmelzpunkt: 234 - 238°C
2.18: 3-[3-Fluor-5-(trifluormethyl)-benzylamino]-piperidin-2,6-dion; Hydrochlorid
   Schmelzpunkt: 241 - 243°C
2.19: 3-(3-Fluorbenzylamino)-piperidin-2,6-dion; Hydrochlorid
   Schmelzpunkt: 231 - 235°C
2.20: 3-(3-Methylbenzylamino)-piperidin-2,6-dion; Hydrochlorid
   Schmelzpunkt: 240 - 242°C
2.21: 3-(4-Trifluormethylbenzylamino)-piperidin-2,6-dion; Hydrochlorid
   Schmelzpunkt: 252 - 255°C
2.22: 3-[4-Fluor-2-(trifluormethyl)-benzylamino]-piperidin-2,6-dion; Hydrochlorid
   Schmelzpunkt: ab 241°C unter Zersetzung
2.23: 3-(4-Fluorbenzylamino)-piperidin-2,6-dion; Hydrochlorid
   Schmelzpunkt: 241 - 242°C
2.24: 3-(4-tert-Butylbenzylamino)-piperidin-2,6-dion;Hydrochlorid
   Schmelzpunkt: ab 239°C unter Zersetzung
2.25: 3-(3,5-Dimethylben2ylamino)-piperidin-2,6-dion; Hydrochlorid
   Schmelzpunkt: ab 226°C unter Zersetzung
2.26: 3-(3-Chlorbenzylamino)-piperidin-2,6-dion; Hydrochlorid
   Schmelzpunkt: 237- 238°C
2.27: 3-(4-Methoxybenzylamino)-piperidin-2,6-dion; Hydrochlorid
   Schmelzpunkt: ab 227°C unter Zersetzung
2.28: 3-(2,4-Dichlorbenzylamino)-piperidin-2,6-dion; Hydrochlorid
   Schmelzpunkt: 240 - 242°C
2.29: 3-(2-Fluorbenzylamino)-piperidin-2,6-dion; Hydrochlorid
   Schmelzpunkt: 245 - 247°C
2.30: 3-(2-Brombenzylamino)-piperidin-2,6-dion; Hydrochlorid
   Schmelzpunkt: 244 - 246°C
2.31: 3-[2-Fluor-5-(trifluormethyl)-benzylamino-piperidin-2,6-dion; Hydrochlorid
   Schmelzpunkt: ab 251°C unter Zersetzung
2.32: 3-(2,3-Dichlorbenzylamino)-piperidin-2,6-dion; Hydrochlorid
   Schmelzpunkt: 246 - 248°C
2. 33 : 3-(3,4-Dichlorbenzylamino)-piperidin-2,6-dion; Hydrochlorid
   Schmelzpunkt: 252 - 254°C
2.34: 3-[3,5-Bis-(trifluormethyl)-benzylamino]-piperidin-2,6-dion; Hydrochlorid
   Schmelzpunkt: 263 - 265°C
2.35: 3-(3-Brombenzy.lamino)-piperidin-2,6-dion; Hydrochlorid
   Schmelzpunkt: 229 - 232°C
2.36: 3-(4-Trifluormethoxybenzylamino)-piperidin-2,6-dion; Hydrochlorid
   Schmelzpunkt: 253 - 255°C
2.37: 3-(4-Chlorbenzylamino)-piperidin-2,6-dion; Hydrochlorid
   Schmelzpunkt: 262 - 265°C
2.38: 3-(4-Methylbenzylamino)-piperidin-2,6-dion; Hydrochlorid
   Schmelzpunkt: 256°C unter Zersetzung
2.39: 3-(2-Ethoxybenzylamino)-piperidin-2,6-dion; Hydrochlorid
   Schmelzpunkt: 208 - 212°C
2.40: 3-(2,5-Dichlorbenzylamino)-piperidin-2,6-dion; Hydrochlorid
   Schmelzpunkt: 242 - 246°C
2.41: 3-(3-Methoxybenzylamino)-piperidin-2,6-dion; Hydrochlorid
   Schmelzpunkt: 217 - 219°C

Alle unter 2.1 bis 2.41 aufgeführten Verbindungen liegen in Form des Racemats vor.

### Beispiel 3

### 3-(3-Amino-benzylamino)-piperidin-2,6-dion: Hydrochlorid

0,56 g des Produkts aus Beispiel 2.14 in einem Gemisch aus 17 ml Essigsäureethylester und 0,85 ml 6N Salzsäure wurden bei 20°C und einem Druck von 4 bar über 0,17 g Palladium auf Aktivkohle (10 % Pd) hydriert. Nach Aufnahme der theoretischen Menge Wasserstoff wurde vom Katalysator abfiltriert und das Filtrat im Vakuum eingedampft. Nach Umkristallisation des Rückstands aus Methanol erhielt man 0,25 g (50 % der Theorie) der razemischen Titelverbindung in Form leicht gefärbter Kristalle, die bei 236 - 239°C schmolzen.
¹H-NMR (DMSO-d₆) : 2, 05 - 2,20 (m, 1H) : 2,28 - 2, 39 (m, 1H); 2,55 - 2,74 (m, 2H); 3,97 - 4,12 (q, 2H); 4,18 - 4,28 (m, 1H) ; 6,58 - 6,70 (m, 3H); 7, 02 - 7,11 (m, 1H) .

### Beispiel 4

Unter Anwendung der in Beispiel 1, Stufe 2, beschriebenen Verfahrensweise und unter Einsatz entsprechender Arylalkylamine wurden analog erhalten:
4.1: 3-Phenethylamino-piperidin-2,6-dion; Hydrochlorid
   Schmelzpunkt: ab 220°C unter Zersetzung
4.2: 3-[2-(2-chlorphenyl)-ethylamino-piperidin-2,6-dion; Hydrochlorid
   Schmelzpunkt: 230°C (Zersetzung)
4.3: 3-(4-Phenylbutylamino)-piperidin-2,6-dion; Hydrochlorid
   Schmelzpunkt: ab 231°C unter Zersetzung
4.4: 3-(N-Benzyl-N-methylamino)-piperidin-2,6-dion; Base
   Schmelzpunkt: 95 - 115°C
4.5: 3-(Methyl-naphth-1-ylmethylamino)-piperidin--2,6-dion; Base
   Schmelzpunkt: 157 - 162°C
   Alle unter 4.1 bis 4.5 aufgeführten Verbindungen liegen in racemischer Form vor.
4.6: (2S)-[(3S) oder (3R)-(2,6-Dioxo-piperidin-3-ylamino)]-phenylessigsäuremethylester; Hydrochlorid
   Schmelzpunkt: 200 - 207°C
4.7: (2R)-[(3S) oder (3R)-(2,6-Dioxo-piperidin-3-ylamino)]-phenylessigsäuremethylester; Hydrochlorid
   Schmelzpunkt: 171 - 177°C (Zersetzung)
4.8: (2S)-[(3R,S)-(2,6-Dioxo-piperidin-3-ylamino)]-3-phenylpropionsäuremethylester; Hydrochlorid (Diastereomerengemisch)
   Schmelzpunkt: 146 - 150°C (Zersetzung)

### Beispiel 5

### 3-Benzylamino-piperidin-2,6-dion

A) Eine Lösung von 0, 50 g 3-Amino-piperidin-2,6-dion [K. Fickentscher, Arch. Pharm. 1974, 307, 840-844], 1,5 ml Triethylamin und 0,4 ml Benzylbromid wurde 20 h bei 20°C gerührt. Dann wurde eingedampft, der Rückstand in 50 ml wässriger Kaliumcarbonatlösung (10 % K₂CO₃) aufgenommen und die Lösung zweimal mit je 40 ml Essigsäureethylester extrahiert. Die organischen Phasen wurden mit je 50 ml dest. Wasser und gesättigter Natriumchloridlösung gewaschen, über Natriumsulfat getrocknet und im Vakuum eingeengt. Der Rückstand wurde durch Flashchromatographie an Kieselgel mit einem Gemisch Essigsäureethylester/Cyclohexan (2/1), das 1 % Triethylamin enthielt, als Elutionsmittel gereinigt, wobei 0,21 g (26 % der Theorie) der Titelverbindung als viskoses Öl anfielen.
   Die Titelverbindung konnte in Form des Hydrobromids als reines S-Enantiomer auch auf folgendem Weg erhalten werden:
B) Stufe 1:

### (2S)-(N-Benzyl-N-benzyloxycarbonylamino)-4-carbamoylbuttersäure

0,95 g (2S)-Benzylamino-4-carbamoylbuttersäure [E. Davidov et al., Isr. J. Chem. 1969, 7, 487-489], gelöst in 4 ml 2 M wässriger Natriumhydroxid- und 8 ml 1 M Natriumhydrogencarbonatlösung, wurden bei 20°C unter Rühren innerhalb von 2,5 h tropfenweise mit 0,6 ml Chlorameisensäurebenzylester versetzt. Dann wurde zweimal mit je 20 ml Diethylether extrahiert. Die wässrige Phase wurde mit conc. Salzsäure auf pH 2 - 3 angesäuert und zweimal mit je 30 ml Essigsäureethylester extrahiert. Die Extrakte wurden mit dest. Wasser gewaschen, über Natriumsulfat getrocknet und im Vakuum eingedampft. Nach Versetzen des öligen Rückstands mit Diethylether fielen 0,55 g (37 % der Theorie) der Titelverbindung in Form farbloser Kristalle an, die bei 98 - 99°C schmolzen.

### Stufe 2:

### (3S)-(N-Butyl-N-benzyloxycarbonylamino]-piperidin-2,6-dion

Zu einer Lösung von 0, 37 g des Produkts aus Stufe 1 in 2,5 ml trockenem Tetrahydrofuran tropfte man eine Lösung von 0,162 g N.N'-Carbonyldiimidazol in 3 ml trockenem Tetrahydrofuran. Es wurde 3,5 h zum Rückfluß erhitzt, dann noch 3 h bei 20°C gerührt. Das nach Abdampfen des Lösungsmittels im Vakuum zurückbleibende Öl wurde in Essigsäureethylester gelöst und die Lösung nacheinander mit je 20 ml 1 M wässriger Natriumhydrogencarbonatlösung, gesättigter Natriumchloridlösung und dest. Wasser gewaschen. Dann wurde über Natriumsulfat getrocknet und im Vakuum eingedampft. Dabei blieben 0,23 g (65 % der Theorie) der Titelverbindung in Form von Kristallen zurück, die bei 51 - 52°C schmolzen.

### Stufe 3:

### (3S)-Benzylamino-piperidin-2,6-dion; Hydrobromid

Die Lösung von 0,15 g des Produkts aus Stufe 2 in 3 ml einer Lösung von Bromwasserstoff in Essigsäure (33 % HBr) wurde 1 h bei 20°C gerührt. Dann wurde das Reaktionsgemisch auf 50 ml Diethylether gegossen. Der sich dabei bildende Niederschlag wurde abgetrennt, mit Diethylether gewaschen und im Vakuum getrocknet. Es blieben 0,08 g (63 % der Theorie) der Titelverbindung in Form von Kristallen zurück, die bei 228 - 230°C unter Zersetzung schmolzen.
¹H-NMR (DMSO-d₆): 2,01 - 2,43 (m, 2H); 2,60 - 2,80 (m 2H); 4,20 - 4,45 (m, 3H); 7,40 - 7,60 (m, 5H).

### Beispiel 6

### 6.1 2-[(3S)-(2,6-Dioxo-piperidin-3-ylamino)methyl]-benzoesäure, Hydrobromid

### Stufe 1:

### 2-[(1S)-(3-Carbamoyl-1-carboxypropylamino)methyl]-benzoesäure

Zu einer Lösung von 1,46 g L-Glutamin in 5 ml einer 2 M wässrigen Natriumhydroxidlösung wurde eine Suspension von 1,65 g 2-Formylbenzoesäure in 5 ml Ethanol und 5 ml 2 M Natriumhydroxidlösung gegeben. Nach 1 h Rühren des Gemisches bei 20°C wurde auf 0°C gekühlt und unter kräftigem Rühren innerhalb von 15 min portionsweise mit 0,25 g Natriumborhydrid versetzt. Nach 90 min wurden weitere 0,33 g 2-Formylbenzoesäure und 0,05 g Natriumborhydrid zugegeben. Nach 16 h Rühren bei 20°C wurde die Reaktionsmischung mit conc. Salzsäure auf pH 2 angesäuert und auf 0°C gekühlt. Der entstandene Niederschlag wurde abgetrennt, mit Aceton gewaschen und im Vakuum getrocknet. Es blieben 0,87 g (31 % der Theorie) der Titelverbindung in Form von Kristallen zurück, die bei 132 - 133°C schmolzen.

### Stufe 2:

### 2-{(1S)-[N-Benzyloxycarbonyl-N-(3-carbamoyl-1-carboxypropyl)aminol-methyl]-benzoesäure

Unter Anwendung der in Beispiel 5 B, Stufe 1, beschriebenen Verfahrensweise wurde aus dem Produkt aus Stufe 1 analog die Titelverbindung in Form von Kristallen, die bei 103 - 104°C unter Zersetzung schmolzen, erhalten.

### Stufe 3:

### 2-{(3S)-[N-Benzyloxycarbonyl-N-(2,6-dioxo-piperidin-3-yl)amino]-methyl}-benzoesäure

Unter Anwendung der in Beispiel 5 B, Stufe 2, beschriebenen Verfahrensweise wurde aus dem Produkt aus Stufe 2 analog die Titelverbindung in Form von Kristallen, die bei 71 - 73°C schmolzen, erhalten.

### Stufe 4:

### 2-[(3S)-(2,6-Dioxo-piperidin-3-ylamino)-methyl]-benzoesäure, Hydrobromid

Unter Anwendung der in Beispiel 5B, Stufe 3, beschriebenen Verfahrensweise wurde aus dem Produkt aus Stufe 3 analog die Titelverbindung in Form nahezu farbloser Kristalle erhalten, die bei 158 - 161°C schmolzen.
¹H-NMR (DMSO-d₆) : 2, 00 - 2,25 (m, 1H) ; 2,35 - 2,95 (m, 1H); 2, 60 - 2,80 (m, 2H); 4,35 - 4,50 (m, 1H); 4,50 - 4,70 (m, 2H) ; 7, 50 - 7,75 (m, 3H); 8, 00 - 8,10 (m, 1H).

### 6.2 2-[(3R)-(2,6-Dioxo-piperidin-3-ylamino)methyl]-benzoesäure; Hydrobromid

Unter Ersatz von L- durch D-Glutamin in Beispiel 6.1,Stufe 1, und bei Anwendung der in Beispiel 6.1 beschriebenen Verfahrensweise wurde analog die Titelverbindung in Form von Kristallen erhalten, die bei 148 - 152 °C schmelzen.

### Beispiel 7

### 2-[(3S)-(2,6-Dioxo-piperidin-3-ylamino)methyl]-N,N-diethylbenzamid; Hydrobromid

### Stufe 1:

### (3S)-[N-(2-Diethylcarbamoylbenzyl)-N-benzyloxy-carbanyl]amino-piperidin-2,6-dion

Eine Lösung von 1,00 g des Produkts aus Beispiel 6.1, Stufe 3, 0,27 g N-Methylmorpholin und 0,46 g 2-Chlor,-4,6-dimethoxy-1,3,5-triazin in 7 ml trockenem Tetrahydrofuran wurde 1 h bei 20°C gerührt. Nach Zugabe von 0,19 g Diethylamin wurde das Rühren noch 7 h fortgesetzt. Dann wurde mit Chloroform auf ein Volumen von 50 ml verdünnt und nacheinander mit 25 ml 0,05 N Salzsäure, 25 ml 1 M wässriger Natriumhydrogencarbonatlösung und gesättigter Natriumchloridlösung gewaschen. Die organische Phase wurde über Natriumsulfat getrocknet und im Vakuum eingedampft. Nach Reinigung des Rückstands durch Flashchromatographie an Kieselgel mit Essigsäureethylester/Cyclohexan (9/1) als Elutionsmittel wurden 0,36 g (32 % der Theorie) der Titelverbindung in Form von Kristallen erhalten, die bei 65 - 66°C schmolzen.

### Stufe 2:

### 2-[(3S)-(2,6-Dioxo-piperidin-3-ylamino)methyl]-N,N-diethylbenzamid; Hydrobromid

0,30 g des Produkts aus Stufe 1 wurden wie unter Beispiel 5B, Stufe 3, beschrieben mit 3 ml einer Lösung von Bromwasserstoff in Essigsäure (33 % HBr) zur Reaktion gebracht. Nach ebenfalls analoger Aufarbeitung und Reinigung durch Umkristallisation aus Methanol/Diethylether wurden 0,175 g (66 % der Theorie) der Titelverbindung in Form von Kristallen erhalten, die bei 119 - 120°C schmolzen.
¹H-NMR (DMSO-d₆): 1,06 (t, J = 7,5 Hz, 3H); 1,21 (t, J = 6,9 Hz, 3H); 2,04 - 2,24 (m, 1H) ; 2,28 - 2,46 (m, 2H); 2, 58 - 2,80 (m, 2H); 3,19 (dd, 2H); 3,51 (dd, 2H) ; 4,24 (s, 2H); 4,25 - 4,40 (m, 1H) ; 7,44 (d, 1H) ; 7, 48 - 7,66 (m, 2H; 7,72 (d, 1H).

### Beispiel 8

Bei Ersatz von Diethylamin in Beispiel 7, Stufe 1, durch andere Amine, Ammoniak oder Hydrazin und unter Anwendung der in Beispiel 7 beschriebenen weiteren Vorgehensweise wurden analog erhalten:
8.1: (3S)-[2-(Morpholin-4-carbonyl)benzylamino] - piperidin-2,6-dion; Hydrobromid
   Schmelzpunkt: 133 - 135°C
8.2: {2-[(3S)-(2,6-Dioxo-piperidin-3-ylamino)methyl-benzoylaminol-essigsäuremethylester; Hydrobromid
   Schmelzpunkt: 121 - 123°C
8.3: 2-[(3S)-(2,6-Dioxo-piperidin-3-ylamino)methyl]-benzamid; Hydrobromid
   Schmelzpunkt: 155 - 156°C (Zersetzung)
8.4: 2-[(3S)-(2,6-Dioxo-piperidin-3-ylamino)methyl]-N-ethylbenzamid; Hydrobromid
   Schmelzpunkt: 144 - 146°C
8.5: (3S)-[2-(Pyrrolidin-l-carbonyl)benzylamino]-piperidin-2,6-dion; Hydrobromid
   Schmelzpunkt: 136 - 138°C
8.6: 2-[(3S)-(2,6-Dioxo-piperidin-3-ylamino)methyl]-benzoesäurehydrazid; Hydrobromid
   Schmelzpunkt: 241 - 242°C
8.7: 2-[(3S)-(2,6-Dioxo-piperidin-3-ylamino)methhyl]-N-phenylbenzamid; Hydrobromid
   Schmelzpunkt: 136 - 138°C
8.8: (2R)-{(3S)-2-[(2,6-Dioxo--piperidin-3-ylamino) methyl]-benzoylamino}-phenylessigsäuremethylester; Hydrobromid
   Schmelzpunkt: 149 - 151°C
8.9: (2S)-{(3S)-2-[(2,6-Dioxo-piperidin-3-ylamino) methyl1-benzoylamino}-phenylessigsäuremethylester; Hydrobromid
   Schmelzpunkt: 181 - 182°C
8.10: 2-[(3R)-(2,6-Dioxo-piperidin-3-ylamino)methyl]-N-phenylbenzamid; Hydrobromid
   Schmelzpunkt: 168 - 171°C
8.11: 2-[(3R)-(2,6-Dioxo-piperidin-3-ylamino)methyl]-N,N-diethyl-benzamid; Hydrobromid
   Schmelzpunkt: 128 - 132°C
8.12: 2-[(3H)-(2,6-Dioxo-piperidin-3-ylamino)methyl]-benzamid; Hydrobromid
   Schmelzpunkt: 232 - 233°C

### Beispiel 9

### 9.1: 2-[(3S)-(2,6-Dioxo-piperidin-3-ylamino)-methyl]-benzoesäuremethylester; Hydrobromid

### Stufe 1:

### 2-{(3S)-[N-Benzyloxycarbonyl-N-(2,6-dioxo-piperidin-3-yl)amino]-methyl}-benzoesäuremethylester

Eine Mischung aus 0,60 g des Produkts aus Beispiel 6.1, Stufe 3, und 0,25 g N,N'-Carbonyldiimidazol in 5 ml trockenem Tetrahydrofuran wurde 1,5 h bei 20°C gerührt. Dann gab man 64 µl Methanol hinzu und rührte weitere 40 h bei 20°C. Nach Abdampfen des Lösungsmittels im Vakuum wurde der Rückstand in 80 ml Chloroform aufgenommen und die Lösung mit 1 M Natriumhydrogencarbonatlösung und dest. Wasser gewaschen. Es wurde über Natriumsulfat getrocknet und im Vakuum eingeengt. Nach säulenchromatographischer Reinigung des Rückstands an Kieselgel mit Chloroform/Aceton (94/6) als Elutionsmittel wurden 0,32 g (51 % der Theorie) der Titelverbindung als viskoses Öl erhalten.

### Stufe 2:

### 2-[(3S)-(2,6-Dioxo-piperidin-3-ylamino)-methyl]-benzoesäuremethylester; Hydrobromid

Durch Abspaltung der Benzyloxycarbonyl-Schutzgruppe im Produkt der Stufe 1 auf die in Beispiel 5B, Stufe 3, beschriebene Verfahrensweise wurde analog die Titelverbindung in Form von Kristallen erhalten, die bei 187°C schmolzen.
¹H-NMR (DMSO-d₆):2,07 - 2,30 (m, 1H) ; 2,30 - 2, 48 (m, 1H) ; 2,60 - 2,85 (m, 2H); 3,90 (s, 3H); 4,40 - 4,70 (m, 3H); 7,58 - 7,78 (m, 3H); 8,05 (d, J = 8 Hz, 1H).

### 9.2: 2-[(3S)-(2,6-Dioxo-piperidin-3-ylamino)-methyl]-benzoesäurebenzylester; Hydrobromid

Bei Ersatz von Methanol durch Benzylalkohol in Beispiel 9.1 und unter Anwendung der dort beschriebenen Verfahrensweise wurde analog die Titelverbindung in Form weißer Kristalle erhalten, die bei 175 - 177°C schmolzen.

### Beispiel 10

### 3-Phenylaminomethyl-piperidin-2,6-dion

Eine Lösung von 1,25 g 3-Methylen-piperidin-2,6-dion [M.J. Wanner u. G.-J. Koomen, Tetrahedron Lett. 1992, 33, 1513-1516] in 100 ml Acetonitril wurde mit 30 ml absolutem Trimethylamin und 2, 75 ml frisch destilliertem Anilin versetzt und das Gemisch 16 h bei 80°C gerührt. Nach dem Abkühlen wurden 10 g Kieselgel zugegeben und im Vakuum eingeengt. Der Rückstand wurde durch Flashchromatographie an Kieselgel mit tert-Butylmethylether/Cyclohexan (2/1) als Elutionsmittel gereinigt. Dabei fielen 1,87 g (86 % der Theorie) der Titelverbindung in Form von Kristallen an, die bei 137°C schmolzen.
¹H-MMR (GDCl₃) : 1, 84 - 1,99 (m, 1H) ; 2, 08 - 2,17 (m, 1H) ; 2,49 - 2,64 (m, 1H) ; 2,73 - 2,83 (m, 2H) ; 3,41 - 3,50 (m, 1H) : 3,60 - 3,70 (m, 1H); 6,64 - 6,80 (m, 3H); 7,17- 7,29 (m , 2H).

### Beispiel 11

Bei Ersatz von Anilin in Beispiel 10 durch andere Amine und unter Anwendung der dort beschriebenen Vorgehensweise, wobei gegebenenfalls statt des Lösungsmittelsystems Acetonitril/Triethylamin auch das Gemisch Toluol/Diisopropyl-ethylamin bei einer Reaktionstemperatur von 110°C eingesetzt wurde, ließen sich analog erhalten:
11.1: 3-[(4-Brom-phenylamino)methyl]-piperidin-2,6-dion
   Schmelzpunkt 149 - 150°C
11.2: 3-[(3-Trifluormethyl-phenylamino)methyl]-piperidin-2,6-dion
   Schmelzpunkt: 135 - 138°C
11.3: 3-(Naphhh-1-ylaminomethyl)-piperidin-2,6-dion
   Schmelzpunkt: 145 - 148°C
11.4: 3-(Biphenyl-4-ylaminomethyl)-piperidin-2,6-dion
   Schmelzpunkt: 135 - 138°C
11.5: 3-[(3-Methoxy-phenylamino)methyl]-piperidin-2,6-dion
   viskos
11.6: 3-[(4-Trityl-phenylamino)methyl]-piperidin-2,6-dion
   Schmelzpunkt: 221 - 225°C
11.7: 3-[(2,6-Dioxo-piperidin-3-ylmethyl)amino]-benzoesäureethylester
   viskos
11.8: 3-(Benzylamino-methyl)-piperidin-2,6-dion
   viskos
11.9: 3-[(3-Acetyl-phenylamino)n]ethyl]-piperidin-2,6-dion
   Schmelzpunkt: 129 - 132°C
11.10: 3-[(N-Methyl-N-phenylamino)methyl]-piperidin-2,6-dion
   Schmelzpunkt: 132 - 134°C
11.11: 3-{[(Naphth-1-ylmethyl)amino]-methyl}-piperidin-2, 6-dion
   viskos
11.12: 3-[(2-Methoxy-phenylamino)methyl]-piperidin-2,6-dion
   viskos
11.13: 3-[(4-Methoxy-phenylamino)methyl]-piperidin-2,6-dion
   Schmelzpunkt: 131 - 134°C
11.14: (2S)-[(2,6-Dioxo-piperidin-3-ylmethyl)amino]-3-phenylpropionsäuremethylester
   viskos
11.15: 2-[(2,6-Dioxo-piperidin-3-ylmethyl)amino-benzamid
   Schmelzpunkt: 203 - 206°C
11.16: 3-[(4-Acetyl-phenylamino)methyl]-piperldin-2,6-dion
   Schmelzpunkt: 160°C
11.17: 3-[(3-Benzoyl-phenylamino)methyl]-piperidin-2,6-dion
   Schmelzpunkt: 152 - 158°C
11.18: 4-[{2,6-Dioxo-piperidin-3-ylmethyl)amino-benzoesauremethylester
   Schmelzpunkt: 142 - 144°C

### Beispiel 12

### 3-[(2-Hydroxymethyl-phenylamino)methyl]-piporidin-2,6-dion

### Stufe 1:

### 3-([2-tert-Butyl-dimethyl-silanyloxymethyl)phenylamino] -methyl}-piperidin-2,6-dion

Bei Ersatz von Anilin in Beispiel 10 durch 2-(tert-Butyl-dimethyl-silanyloxymethyl) phenylamin und unter Anwendung der dort beschriebenen Verfahrensweise wurde die Titelverbindung in Form weißer Kristalle erhalten, die bei 85 - 87°C schmolzen.

### Stufe 2:

### 3-[(2-Hydroxymethyl-phenylamino)methyl]-piperidin-2,6-dion

Zu einer Lösung von 0, 20 g des Produkts aus Stufe 1 in 5 ml Tetrahydrofuran wurden 5 ml einer 1 M Lösung von Tetrabutylammoniumfluorid-Trihydrat in Tetrahydrofuran gegeben. Man rührte 3 h bei 20°C, dampfte im Vakuum ein und reinigte den Rückstand durch Flashchromatographie an Kieselgel mit Essigsäureethylester als Elutionsmittel. Dabei wurden 0,12 g (85 % der Theorie) der Titelverbindung in Form eines gelblichen Öls erhalten.

### Beispiel 13

Bei Ersatz von Anilin in Beispiel 10 durch Thiophenole oder Mercaptane und unter Anwendung der dort beschriebenen Verfahrensweise wurden analog erhalten:
13.1: 3-Phenylsulfanylmethyl-piperidin-2,6-dion
   Schmelzpunkt: 98°C
13.2: 3-Phenethylsulfanylmethyl-piperidin-2,6-dion
   Schmelzpunkt: 78°C
13.3: 2-(2,6-Dioxo-piperidin-3-ylmethyl)sulfanyl)-benzoesäuremethylester
   Schmelzpunkt: 142 - 144°C
13.4: 3-Benzylsulfanylmethyl-piperidin-2,6-dion
   Schmelzpunkt: 105 - 107°C
13.5: 3-(3-Amino-phenylsulfanylmethyl)-piperidin-2,6-dion
   Schmelzpunkt: 133 - 135°C
13.6: 2-(2,6-Dioxo-piperidin-3-ylmethylsulfanyl)-6-nitro-benzoesäuremethylester
   Schmelzpunkt: 147 - 150°C

### Beispiel 14

### 2-Amino-6-(2,6-dioxo-piperidin-3-ylmethylsulfanyl)-bonzoesäuremethylester

Die Titelverbindung wurde durch katalytische Hydrierung des Produkts aus Beispiel 13.6 über Palladium auf Aktivkohle (10 % Pd) unter den in Beispiel 3 beschriebenen Bedingungen analog erhalten.
Schmelzpunkt: 164 - 167°C

### Beispiel 15

### 3-Phenylsulfanylmethyl-1-piperidin-1-ylmethyl-piperidin-2,6-dion

Eine Lösung von 1,20 g des Produkts aus Beispiel 13.1 in 30 ml Ethanol wurde mit 0,52 ml wässriger Formaldehydlösung (35 %) und 0,43 ml Piperidin versetzt. Nach einstündigem Erhitzen des Gemisches unter Rückfluß wurde im Vakuum eingedampft. Der Rückstand wurde in Essigsäureethylester aufgenommen und die Lösung solange mit n-Hexan versetzt, bis sich ein kristalliner Niederschlag bildete. Dieser wurde abgetrennt und im Vakuum getrocknet. Dabei fielen 1,23 g (74 % der Theorie) der Titelverbindung an, die einen Schmelzpunkt von 63 - 66°C aufwiesen.
¹H-NMR (DMSO-d₆) : 1,37 - 1,47 (m, 6H), 1,72 - 1,88 (m, 1H), 2,08 - 2,16 (m, 1H), 2,21 - 2,33 (m, 4H), 2,49 - 2,57 (m, 1H), 2,70 - 2,82 (m, 1H), 3,07 - 3,18 (m, 1H) , 3,28 - 3,33 (m, 1H), 3,47 - 3,56 (m, 1H) , 4,56 - 4,69 (m, 2H), 7, 17 - 7, 25 (m, 1H), 7,28 - 7,39 (m, 4H).

### Stimulation humaner Monozyten mit Lipopolysaccharid zur Sekretion von IL-12

Humane Monozyten wurden aus peripheren Blut-mononucleären Zellen (PBMC), die mittels einer Ficoll-Dichtegradientenzentrifugation von heparinisiertem Vollblut gewonnen wurden, isoliert. Dazu wurden die PBMC mit einem monoklonalen Antikörper inkubiert, der gegen das Monozyten-spezifische oberflächenmolekül CD14 gerichtet ist und an den superparamagnetische Microbeads (Miltenyi Biotech, Bergisch Gladbach) gekoppelt sind. Zur positiven Selektion der markierten Monozyten aus dem Zellgemisch der PBMC wurde die Gesamtzellsuspension auf eine Säule mit ferromagnetischer Trägermatrix aufgebracht und diese in ein Magnetfeld gestellt. Dadurch wurden die Zellen, die mit Microbeads beladen waren, an die Trägermatrix gebunden, unmarkierte Zellen passierten die Säule und wurden verworfen. Nach Herausnehmen der Matrix aus dem Magnetfeld wurden die Antikörper-beladenen Zellen durch Spülen der nun entmagnetisierten Säule mit Puffer eluiert. Die Reinheit dieser so erhaltenen CD14-positiven Monozytenpopulation betrug etwa 95 bis 98%. Diese Monozyten wurden in einer Dichte von 10⁶ Zellen/ml Kulturmedium (RPMI, supplementiert mit 10% foetalem Kälberserum) mit den in DMSO-gelösten Prüfsubstanzen für eine Stunde bei 37°C und 5% CO₂ inkubiert. Anschließend wurden 20 µg/ml LPS aus E. coli zugegeben.

Nach 24 Stunden wurden zellfreie Kulturüberstände genommen und auf den Gehalt an IL-12 getestet.

Die Konzentration von IL-12 in den Zellkulturüberständen wurde mittels Sandwich-ELISAs unter Verwendung zweier anti-IL-12 monoklonaler Antikörper (Biosource Europe, Fleurus, Belgien) bestimmt. Eine Referenzstandardkurve mit humanem IL-12 wurde eingeschlossen. Das Detektionslimit des IL-12 ELISAs betrug 10 pg/ml.

**Tabelle 1. Einfluß der Prüfsubstanzen auf die IL-12-Produktion von LPS-aktivierten Monozyten.**

| Beispiel-Nr. | Hemmung der IL-12 Produktion | |
|---|---|---|
| | Maximal (%) | IC50 (µg/ml) |
| 6.1 | 85 | 1,0 |
| 6.2 | 75 | 1,0 |
| 9.1 | 90 | 0,1 |
| 9.2 | | |
| 8.3 | 90 | 0,15 |
| 8.12 | 84 | 1,0 |
| 7 | 90 | 1,5 |
| 8.11 | 90 | 0,2 |
| 8.1 | 90 | 1,8 |
| 8.5 | 80 | 2,0 |
| 8.4 | 80 | 0,9 |
| 8.7 | 55 | 0,7 |
| 3.10 | 50 | - |
| 8.6 | 90 | 0,04 |
| 8.2 | 70 | 1,8 |
| 13.3 | 50 | 6,0 |
| 13.6 | 57 | 3,0 |

Die in Tabelle 1 dargestellten Ergebnisse zeigen, daß die substituierten Glutarimide immunmodulatorisch wirksam sind. Sie hemmen die Synthese von IL-12 durch LPS-aktivierte Monozyten in potenter Weise.

## Patentansprüche

1. Substituierte Glutarimide der allgemeinen Formel I 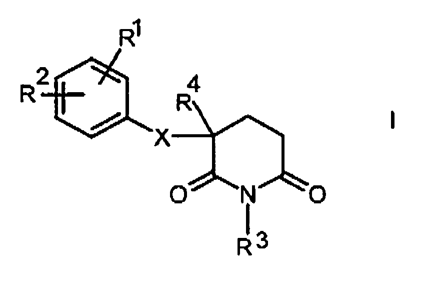 in der
X eine Gruppe der Formel (CH₂)ₙ-(CR⁸R⁹)ₚ-Z-(CR⁸R⁹)ₘ bedeutet,
Z für ein Schwefel- oder Sauerstoffatom, die SO- oder SO₂-Gruppe, den Rest NR⁸ (gegebenenfalls in Form des N-oxids) oder eine CR⁸R⁹-Gruppe,
m und p für 0 oder 1,
n für 0, 1, 2 oder 3 stehen, wobei m, n und p nicht gleichzeitig 0 sein können,
R¹ und R² gleich oder verschieden sind und für die Carboxylgruppe, eine Estergruppe der Formel CO0R⁵ oder eine Acylgruppe der Formel COR⁵, in denen R⁵ jeweils eine Alkylgruppe (geradkettig oder verzweigt) mit 1 bis 6 C-Atomen (gegebenenfalls substituiert mit dem Rest CO0R⁵ und/oder einer Phenylgruppe), eine C₃- bis C₇-Cycloalkylgruppe oder einen Phenyl- oder Benzylrest bedeutet, oder eine Amidgruppe der Formel CONR⁶R⁷, in der R⁶ und R⁷ gleich oder verschieden sind und Wasserstoff, eine Alkylgruppe (geradkettig oder verzweigt) mit 1 bis 6 C-Atomen (gegebenenfalls substituiert mit dem Rest COOR⁵ und/oder einer Phenylgruppe), den Allylrest, den Phenylrest oder zusammengenommen mit dem N-Atom die Hydrazidgruppe, den Pyrrolidin-, Piperidin-, Hexamethylenimin-, Morpholin-, Thiomorpholin-, Piperazin- oder N-Methylpiperazinring darstellen, für Wasserstoff, Brom, Chlor, Fluor, eine Mono-, Di- oder Trifluormethyl-, Trityl-, Hydroxy-, Hydroxymethyl-, Trifluormethoxy-, Nitro-, Amino- (gegebenenfalls substituiert mit dem Rest CH(=O) oder COR⁵ oder einer Alkylsulfonylgruppe) oder Dimethylaminogruppe, einen Alkyl- oder Alkoxyrest (geradkettig oder verzweigt) mit 1 bis 6 C-Atomen, einen Amidinrest der Formel NH-CH(=NH) oder NH- C(=NH)R⁵, einen Phenylrest oder einen ankondensierten Benzolring (jeweils gegebenenfalls substituiert mit vorgenannten Atomen oder Gruppen, d.h. mit Wasserstoff, Brom, Chlor, Fluor, eine Mono-, Di- oder Trifluormethyl-, Trityl-, Hydroxy-, Hydroxymethyl-, Trifluormethoxy-, Nitro-, Amino- (gegebenenfalls substituiert mit dem Rest CH(=O)oder COR⁵ oder einer Alkylsulfonylgruppe) oder Dimethylaminogruppe, einen Alkyl- oder Alkoxyrest (geradkettig oder verzweigt) mit 1 bis 6 C-Atomen, einen Amidinrest der Formel NH-CH(=NH) oder NH- C(=NH)R⁵), mit der Einschränkung, daß für den Fall Z=CR⁸R⁹ R¹ und R² nicht gleichzeitig Wasserstoff und für den Fall Z = S und m = 0 nicht die Methoxygruppe darstellen können,
stehen,
R³ für Wasserstoff, den Hydroxyrest oder eine Gruppe der Formel CH₂-NR⁶R⁷, in der R⁶ und R⁷ wie oben definiert sind, steht,
R⁴ für Wasserstoff, eine C₁ bis C₃-Alkylgruppe, ein Fluoratom, die Difluor- oder Trifluormethylgruppe steht,
R⁸ für Wasserstoff, eine Alkylgruppe mit 1 bis 4 C-Atomen (geradkettig oder verzweigt), den Benzyl- oder Phenethylrest (gegebenenfalls substituiert mit vorgenannten Atomen oder Gruppen, d.h. mit Wasserstoff, Brom, Chlor, Fluor, eine Mono-, Di- oder Trifluormethyl-, Trityl-, Hydroxy-, Hydroxymethyl-, Trifluormethoxy-, Nitro-, Amino- (gegebenenfalls substituiert mit dem Rest CH(=O)oder COR⁵ oder einer Alkylsulfonylgruppe) oder Dimethylaminogruppe, einen Alkyl- oder Alkoxyrest (geradkettig oder verzweigt) mit 1 bis 6 C-Atomen, einen Amidinrest der Formel NH-CH(=NH) oder NH- C(=NH)R⁵) steht,
und R⁹ dieselbe Bedeutung wie R⁸ hat, für die Estergruppe der Formel COOR⁵, den Phenylrest, die Hydroxygruppe oder einen Alkoxyrest (geradkettig oder verzweigt) mit 1 bis 4 C- Atomen, ein Fluor- oder Chloratom oder die Trifluormethylgruppe steht,
und deren Enantiomere, Enantiomerengemische, Racemate, Diastereomere oder Diastereomerengemische in Form ihrer Basen oder Salze physiologisch verträglicher Säuren.

2. Substituierte Glutarimide der allgemeinen Formel I gemäß Anspruch 1, **dadurch gekennzeichnet, daß** X für die Gruppen CH₂-N und S-CH₂ steht, R¹ für die Carboxylgruppe, eine Estergruppe der Formel COOR⁵ wie oben definiert, eine Acylgruppe der Formel COR⁵ wie oben definiert oder eine Amidgruppe der Formel CONR⁶R⁷, in der R⁶ und R⁷ gleich oder verschieden sind und Wasserstoff, eine Alkylgruppe (geradkettig oder verzweigt) mit 1 bis 6 C-Atomen (gegebenenfalls substituiert wie oben definiert),den Phenylrest oder zusammengenommen mit dem N-Atom die Hydrazidgruppe, den Pyrrolidin- oder Morpholidinring darstellen, steht, R² für Wasserstoff, die Nitro- oder Aminogruppe, R³ für Wasserstoff und R⁴ für Wasserstoff, Methyl oder Fluor stehen.

3. Verbindungen nach Anspruch 1:
2-[(3S)-(2,6-Dioxo-piperidin-3-ylamino)methyl]-benzoesäure
2-[(3R)-(2,6-Dioxo-piperidin-3-ylamino)methyl]-benzoesäure
2-[(3S)-(2,6-Dioxo-piperidin-3-ylamino)methyl]-N,N-diethylbenzamid
(3S)-[2-(Morpholin-4-carbonyl)benzylamino]-piperidin 2,6-dion
{2-[(3S)-(2,6-Dioxo-piperidin-3-ylamino)methyl] benzoylamino}-essigsäuremethylester
2-[(3S)-(2,6-Dioxo-piperidin-3-ylamino)methyl]-benzamid
2-[(3S)-(2,6-Dioxo-piperidin-3-ylamino)methyl]-N-ethylbenzamid
(3S)-[2-(Pyrrolidin-2-carbonyl)benzylamino]-piperidin-2,6-dion
2-[(3S)-(2,6-Dioxo-piperidin-3-ylamino)methyl]-benzoesäurehydrazid
2-[(3S)-(2,6-Dioxo-piperidin-3-ylamino)methyl]-N-phenylbenzamid
2-[(3R)-(2,6-Dioxo-piperidin-3-ylamino)methyl]-N-phenylbenzamid
2-[(3R)-(2,6-Dioxo-piperidin-3-ylamino)methyl]-N,N-diethyl-benzamid
2-[(3R)-(2,6-Dioxo-piperidin-3-ylamino)methyl]-benzamid
2-[(3S)-(2,6-Dioxo-piperidin-3-ylamino)-methyl] - benzoesäuremethylester
2-[(3S)-(2,6-Dioxo-piperidin-3-ylamino)-methyl]-benzoesäurebenzylester
2-(2,6-Dioxo-piperidin-3-ylmethyl)sulfanyl)-benzoesäuremethylester
2-(2,6-Dioxo-piperidin-3-ylmethylsulfanyl)-6-nitro-benzoesäuremethylester.

4. Arzneimittel enthaltend als Wirkstoff wenigstens eine Verbindung nach Anspruch 1.

5. Arzneimittel nach Anspruch 4 mit immunmodulatorischer wirkung und/oder zur Behandlung von Angiopathien und/oder hämatalogisch/onkologischen Erkrankungen.

6. Verfahren zur Herstellung von substituierten Glutarimiden der allgemeinen Formel I nach Anspruch 1, in der R³ für Wasserstoff oder den Hydroxyrest steht, **dadurch gekennzeichnet, daß** Glutarsäurederivate der allgemeinen Formel II, 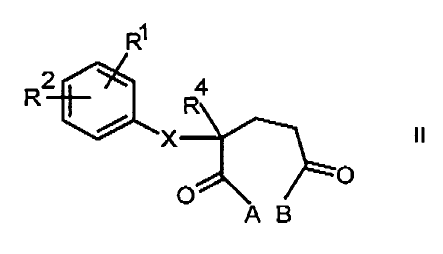
in der X, R¹, R² und R⁴ dieselben Bedeutungen wie oben haben, entweder wenn A für OH und B für NH₂ oder NHOH oder umgekehrt stehen, in Gegenwart aktivierender Reagenzien, vorzugsweise Carbonyldiimidazol, cyclisiert werden oder
wenn A und B gleich OH sind, in Acetanhydrid erhitzt werden und die durch Ringschluß erhaltenen Anhydride durch weiteres Erhitzen mit Harnstoff oder einer anderen Stickstoffquelle zu Verbindungen der Formel I mit R³ = H umgesetzt werden.

7. Verfahren zur Herstellung von substituierten Glutarimiden der allgemeinen Formel I nach Anspruch 1, in der R³ = H ist, **dadurch gekennzeichnet, daß** Lactame der allgemeinen Formel III 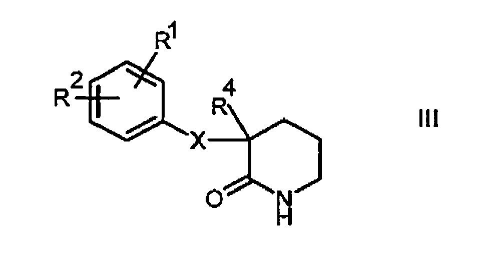 in der R¹, R², R⁴ und X dieselben Bedeutungen wie oben haben, zum entsprechenden Imid oxidiert werden, vorzugsweise mit m-Chlor-perbenzoesäure oder Ruthenium (IV) oxid/Natriumperiodat.

8. Verfahren zur Herstellung von substituierten Glutarimiden der allgemeinen Formel I nach Anspruch 1, in der R³ für CH₂-NR⁶R⁷ steht, **dadurch gekennzeichnet, daß** Verbindungen der Formel I mit R³= H mit Paraformaldehyd oder einer wässrigen Formaldehydlösung und einem sekundären Amin der Formel HNR⁶R⁷, wobei R⁶ und R⁷ wie oben definiert sind, umgesetzt werden.

9. Verfahren zur Herstellung von substituierten Glutarimiden der allgemeinen Formel I nach Anspruch 1, in der R⁴ eine C₁ bis C₃-Alkylgruppe, ein Fluoratom, die Difluor- oder Trifluormethylgruppe bedeutet, **dadurch gekennzeichnet, daß** in Verbindungen der Formel I mit R⁴- H dieser Wasserstoff durch an sich bekannte Alkylierungsreaktionen gegen eine C₁ bis C₃-Alkylgruppe oder die Difluor- oder die Trifluormethylgruppe oder durch an sich bekannte Halogenierungsreaktionen gegen ein Fluoratom, ausgetauscht wird.

10. Verfahren zur Herstellung von substituierten Glutarimiden der allgemeinen Formel I nach Anspruch 1, in der für die Gruppe X m=0 und Z=NR⁸ ist, wobei R⁸, und n und p dieselben Bedeutungen wie oben haben, **dadurch gekennzeichnet, daß** α-Aminoglutarimide der allgemeinen Formel IV, 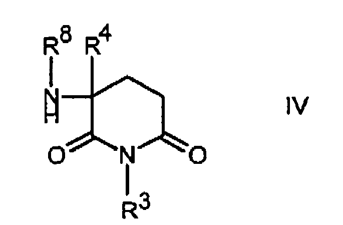 in der R³, R⁴ und R⁸ dieselben Bedeutungen wie oben haben, mit Verbindungen der allgemeinen Formel V, 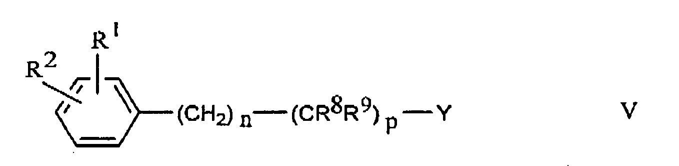 in der R¹, R², R⁸, R⁹, n und p dieselben Bedeutungen wie oben haben und Y für ein Chlor-, Brom- oder Iodatom oder den Toluol-4-Sulfonatrest steht, alkyliert werden.

11. Verfahren zur Herstellung von substituierten Glutarimiden der allgemeinen Formel I nach Anspruch 1, in der für die Gruppe X p=1 und R⁸ oder R⁹ Wasserstoff ist, **dadurch gekennzeichnet, daß** sie durch reduktive Aminierung aus Verbindungen der allgemeinen Formeln VI und IV 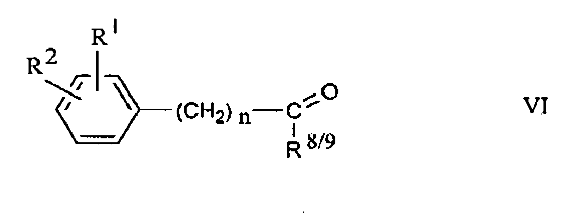 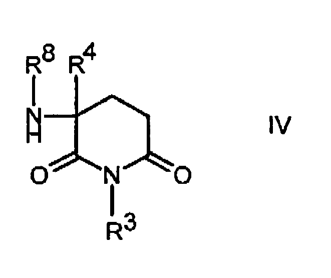 in denen R¹, R², R⁴, R⁸, R⁹ und n dieselben Bedeutungen haben wie oben definiert und R³ für Wasserstoff oder die Hydroxygruppe steht, hergestellt werden, wobei als Reduktionsmittel vorzugsweise Natriumborhydrid, Natriumtriacetoxyborhydrid, Natriumcyanoborhydrid, der Boran-Pyridinkomplex oder katalytisch angeregter Wasserstoff eingesetzt werden.

12. Verfahren zur Herstellung von substituierten Glutarimiden der allgemeinen Formel I nach Anspruch 1, in der für die Gruppe X m-0 und Z für O, S oder NR⁶ steht und R⁸, n und p wie oben definiert sind, **dadurch gekennzeichnet, daß** eine Verbindung der allgemeinen Formel VII, 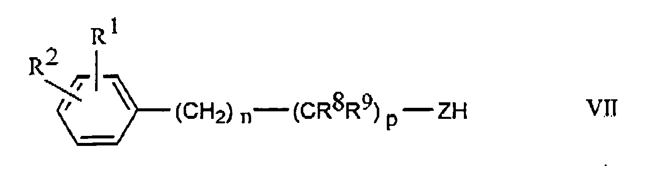 in der R¹, R² dieselben Bedeutungen wie oben haben , mit α-Bromglutarimiden der allgemeinen Formel VIII, 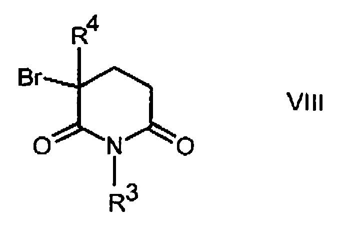 in der R³ und R⁴ wie oben definiert sind, alkyliert wird.

13. Verfahren zur Herstellung von substituierten Glutarimiden der allgemeinen Formel I nach Anspruch 1, in der R⁴=H ist und in der Gruppe X n und p wie oben definiert sind, m für 1, Z für O, S oder NR⁸ und in der Gruppe CR⁸R⁹ mindestens einer der Reste R⁸ oder R⁹ für Wasserstoff stehen, **dadurch gekennzeichnet, daß** eine Verbindung der allgemeinen Formel VII an ein 3-Methylenglutarimid der allgemeinen Formel IX addiert wird, 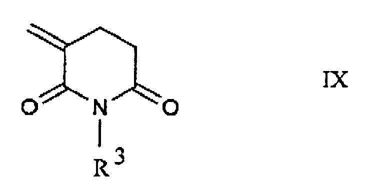 wobei die Umsetzung vorzugsweise in Lösungsmitteln wie Acetonitril oder Toluol unter Zusatz von tertiären Aminen wie Triethylamin oder Diisopropyl-ethylamin bei Temperaturen von 80 - 110°C erfolgt.

14. Verfahren zur Herstellung von substituierten Glutarimiden der allgemeinen Formel I nach Anspruch 1, in der R¹ und/oder R² für eine Aminogruppe stehen,**dadurch gekennzeichnet, daß** sie durch Reduktion von Verbindungen der Formel I mit R¹ und/oder R² = NO₂ erhalten werden, wobei die Reduktion durch katalytisch angeregten Wasserstoff in säurehaltigen organischen Lösungsmitteln wie Essigsäureethylester unter bevorzugter Verwendung von Palladiumkatalysatoren oder aber mit Metallen wie Zinn oder Eisen in saurer Lösung erfolgt.

15. Verfahren zur Herstellung von substituierten Glutarimiden der allgemeinen Formel I nach Anspruch 1, in der Z in der Gruppe X für SO oder SO₂ steht, **dadurch gekennzeichnet, daß** sie durch stufenweise Oxidation des entsprechenden Thioethers (Z = S) erhalten werden, wobei als Oxidationsmittel Wasserstoffperoxid in essigsaurer Lösung, m-Chlorperbenzoesäure, tert-Butylhydroperoxid oder Oxone, letzteres bevorzugt zur Herstellung der Sulfone (Z = SO₂), eingesetzt werden, oder die Oxidation zu Sulfoxiden (Z = SO) alternativ asymmetrisch gestaltet wird durch Einsatz des Sharpless-Systems oder des Davis-Reagenz oder über enzymatische Methoden.

16. Verfahren zur Herstellung von substituierten Glutarimiden der allgemeinen Formel I nach Anspruch 1, in der Z in der Gruppe X für den Rest NR⁸ steht, **dadurch gekennzeichnet, daß** dieser Rest in das entsprechende N-Oxid überführt wird, wobei bevorzugt Wasserstoffperoxid als Oxidationsmittel eingesetzt wird.

17. Verwendung eines substituierten Glutarimids der Formel I nach Anspruch 1 zur Herstellung eines Arzneimittels.

18. Verwendung nach Anspruch 17 zur Herstellung eines Arzneimittels mit immunmodulatorischer Wirkung.

19. Verwendung nach Anspruch 17 zur Herstellung eines Arzneimittels zur Behandlung von Angiopathien.

20. Verwendung nach Anspruch 17 zur Herstellung eines Arzneimittels zur Behandlung von hämatologisch/onkologischen Erkrankungen.

## Claims

1. Substituted glutarimides of the general formula I 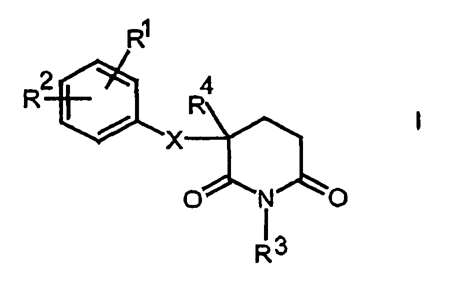 in which
X denotes a group of the formula (CH₂)ₙ-(CR⁸R⁹)ₚ-Z-(CR⁸R⁹)ₘ,
Z represents a sulfur or oxygen atom, the SO or SO₂ group, the NR⁸ radical (optionally in the form of the N-oxide) or a CR⁸R⁹ group,
m and p represent 0 or 1,
n represents 0, 1, 2 or 3, wherein m, n and p cannot simultaneously be 0,
R¹ and R² are the same or different and represent the carboxyl group, an ester group of the formula COOR⁵ or an acyl group of the formula COR⁵, in which R⁵ in each case denotes an alkyl group (straight-chain or branched) having 1 to 6 C atoms (optionally substituted by the radical COOR⁵ and/or a phenyl group), a C₃- to C₇- cycloalkyl group or a phenyl or benzyl radical, or an amide group of the formula CONR⁶R⁷, in which R⁶ and R⁷ are the same or different and represent hydrogen, an alkyl group (straight-chain or branched) having 1 to 6 C atoms (optionally substituted by the radical COOR⁵ and/or a phenyl group), the allyl radical, the phenyl radical or taken together with the N atom represent the hydrazide group or the pyrrolidine, piperidine, hexamethyleneimine, morpholine, thiomorpholine, piperazine or N- methylpiperazine ring, hydrogen, bromine, chlorine, fluorine, a mono-, di- or trifluoromethyl, trityl, hydroxyl, hydroxymethyl, trifluoromethoxy, nitro, amino (optionally substituted by the radical CH(=O) or COR⁵ or an alkylsulfonyl group) or dimethylamino group, an alkyl or alkoxy radical (straight-chain or branched) having 1 to 6 C atoms, an amidine radical of the formula NH-CH(=NH) or NH-C(=NH)R⁵, a phenyl radical or a fused-on benzene ring (optionally substituted in each case by the abovementioned atoms or groups, i.e. by hydrogen, bromine, chlorine, fluorine, a mono-, di- or trifluoromethyl, trityl, hydroxyl, hydroxymethyl, trifluoromethoxy, nitro, amino (optionally substituted by the radical CH(=O) or COR⁵ or an alkylsulfonyl group) or dimethylamino group, an alkyl or alkoxy radical (straight-chain or branched) having 1 to 6 C atoms, an amidine radical of the formula NH-CH(=NH) or NH-C(=NH)R⁵), with the restriction that if Z=CR⁸R⁹ R¹ and R² cannot simultaneously represent hydrogen and if Z = S and m = 0 they cannot represent the methoxy group,
R³ represents hydrogen, the hydroxy radical or a group of the formula CH₂-NR⁶R⁷, in which R⁶ and R⁷ are defined as above,
R⁴ represents hydrogen, a C₁- to C₃-alkyl group, a fluorine atom or the difluoro- or trifluoromethyl group
R⁸ represents hydrogen, an alkyl group having 1 to 4 C atoms (straight-chain or branched), the benzyl or phenethyl radical (optionally substituted by the abovementioned atoms or groups, i.e. by hydrogen, bromine, chlorine, fluorine, a mono-, di- or trifluoromethyl, trityl, hydroxyl, hydroxymethyl, trifluoromethoxy, nitro, amino (optionally substituted by the radical CH(=O) or COR⁵ or an alkylsulfonyl group) or dimethylamino group, an alkyl or alkoxy radical (straight- chain or branched) having 1 to 6 C atoms, an amidine radical of the formula NH-CH(=NH) or NH-C(=NH)R⁵),
and R⁹ has the same meaning as R⁸, represents the ester group of the formula COOR⁵, the phenyl radical, the hydroxyl group or an alkoxy radical (straight-chain or branched) having 1 to 4 C atoms, a fluorine or chlorine atom or the trifluoromethyl group,
and enantiomers, enantiomer mixtures, racemates, diastereomers or diastereomer mixtures thereof in the form of their bases or salts of physiologically acceptable acids.

2. Substituted glutarimides of the general formula I according to claim 1, **characterised in that** X represents the groups CH₂-N and S-CH₂, R¹ represents the carboxyl group, an ester group of the formula COOR⁵ defined as above, an acyl group of the formula COR⁵ defined as above or an amide group of the formula CONR⁶R⁷, in which R⁶ and R⁷ are the same or different and represent hydrogen, an alkyl group (straight-chain or branched) having 1 to 6 C atoms (optionally substituted as defined above), the phenyl radical or which taken together with the N atom represent the hydrazide group or the pyrrolidine or morpholidine ring, R² represents hydrogen or the nitro or amino group, R³ represents hydrogen and R⁴ represents hydrogen, methyl or fluorine.

3. Compounds according to claim 1:
2-[(3S)-(2,6-dioxopiperidin-3-ylamino)methyl]-benzoic acid
2-[(3R)-(2,6-dioxopiperidin-3-ylamino)methyl]-benzoic acid
2-[(3S)-(2,6-dioxopiperidin-3-ylamino)methyl]-N,N-diethylbenzamide
(3S)-[2-morpholine-4-carbonyl)benzylamino]-piperidine-2,6-dione
{2-[(3S)-(2,6-dioxopiperidin-3-ylamino)methyl]-benzoylamino}-acetic acid methyl ester
2-[(3S)-(2,6-dioxopiperidin-3-ylamino)methyl]-benzamide
2-[(3S)-(2,6-dioxopiperidin-3-ylamino)methyl]-N-ethyl-benzamide
(3S)-[2-pyrrolidine-1-carbonyl)benzylamino]-piperidine-2,6-dione
2-[(3S)-(2,6-dioxopiperidin-3-ylamino)methyl]-benzoic acid hydrazide
2-[(3S)-(2,6-dioxopiperidin-3-ylamino)methyl]-N-phenylbenzamide
2-[(3R)-(2,6-dioxopiperidin-3-ylamino)methyl]-N-phenylbenzamide
2-[(3R)-(2,6-dioxopiperidin-3-ylamino)methyl]-N,N-diethylbenzamide
2-[(3R)-(2,6-dioxopiperidin-3-ylamino)methyl]-benzamide
2-[(3S)-(2,6-dioxopiperidin-3-ylamino)methyl]-benzoic acid methyl ester
2-[(3S)-(2,6-dioxopiperidin-3-ylamino)methyl]-benzoic acid benzyl ester
2-(2,6-dioxopiperidin-3-ylmethylsulfanyl)-benzoic acid methyl ester
2-(2,6-dioxopiperidin-3-ylmethylsulfanyl)-6-nitrobenzoic acid methyl ester

4. Medicament containing as an active compound at least one compound according to claim 1.

5. Medicament according to claim 4 with an immunomodulatory action and/or for the treatment of angiopathies and/or haematological/oncological diseases.

6. Process for the preparation of substituted glutarimides of the general formula I according to claim 1, in which R³ represents hydrogen or the hydroxy radical, **characterised in that** glutaric acid derivatives of the general formula II, 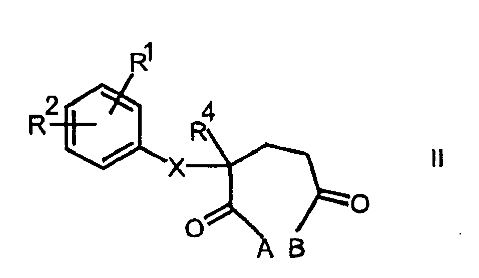
in which X, R¹, R² and R⁴ have the same meanings as above, are either, if A represents OH and B represents NH₂ or NHOH or vice versa, cyclised in the presence of activating reagents, preferably carbonyldiimidazole, or,
if A and B are both OH, heated in acetic anhydride and the anhydrides obtained by cyclisation are reacted to give compounds of the formula I where R³= H by further heating with urea or another nitrogen source.

7. Process for the preparation of substituted glutarimides of the general formula I according to claim 1, in which R³ = H, **characterised in that** lactams of the general formula III 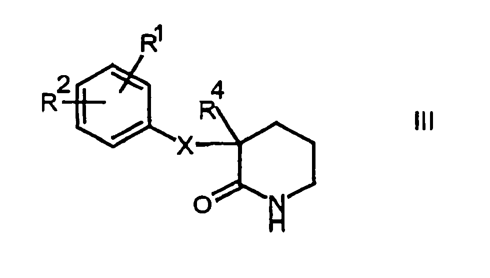 in which R¹, R², R⁴ and X have the same meanings as above, are oxidised to the corresponding imide, preferably with m-chloroperbenzoic acid or ruthenium(IV) oxide/sodium periodate.

8. Process for the preparation of substituted glutarimides of the general formula I according to claim 1, in which R³ represents CH₂-NR⁶R⁷, **characterised in that** compounds of the formula I where R³ = H are reacted with paraformaldehyde or an aqueous formaldehyde solution and a secondary amine of the formula HNR⁶R⁷, wherein R⁶ and R⁷ are defined as above.

9. Process for the preparation of substituted glutarimides of the general formula I according to claim 1, in which R⁴ denotes a C₁- to C₃-alkyl group, a fluorine atom or the difluoro- or trifluoromethyl group, **characterised in that** in compounds of the general formula I where R⁴ = H, this hydrogen is exchanged for a C₁- to C₃-alkyl group or the difluoro- or trifluoromethyl group by alkylation reactions known *per se* or for a fluorine atom by halogenation reactions known *per se.*

10. Process for the preparation of substituted glutarimides of the general formula I according to claim 1, in which for the group X m = 0 and Z = NR⁸, wherein R⁸ and n and p have the same meanings as above, **characterised in that** α-aminoglutarimides of the general formula IV, 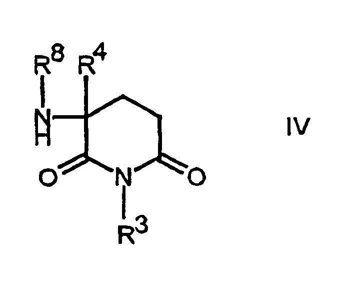 in which R³, R⁴ and R⁸ have the same meanings as above, are alkylated with compounds of the general formula V, 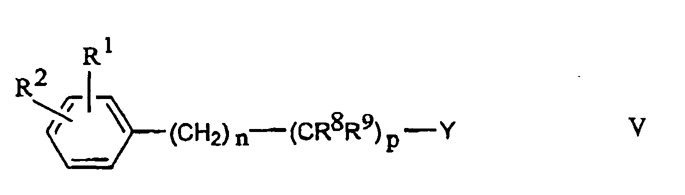 in which R¹, R², R⁸, R⁹, n and p have the same meanings as above and Y represents a chlorine, bromine or iodine atom or the toluene-4-sulfonate radical.

11. Process for the preparation of substituted glutarimides of the general formula I according to claim 1, in which for the group X p = 1 and R⁸ or R⁹ is hydrogen, **characterised in that** they are prepared by reductive amination from compounds of the general formulae VI and IV, 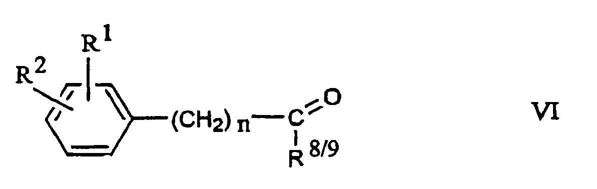 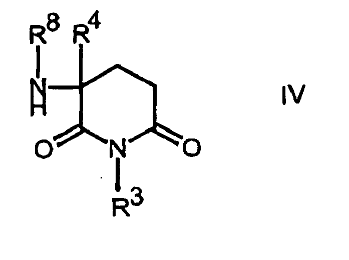 in which R¹, R², R⁴, R⁸, R⁹ and n have the same meanings as defined above and R³ represents hydrogen or the hydroxyl group, sodium borohydride, sodium triacetoxyborohydride, sodium cyanoborohydride, the borane-pyridine complex or catalytically excited hydrogen preferably being employed as the reducing agent.

12. Process for the preparation of substituted glutarimides of the general formula I according to claim 1, in which for the group X m = 0 and Z represents O, S or NR⁸ and R⁸, n and p are defined as above, **characterised in that** a compound of the general formula VII, 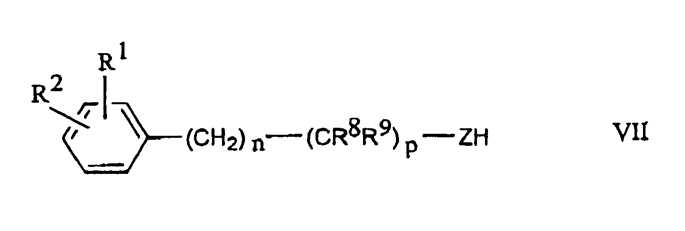 in which R¹ and R² have the same meanings as above, is alkylated with α-bromoglutarimides of the general formula VIII, 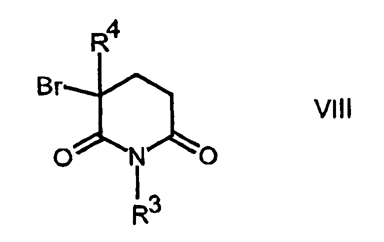 in which R³ and R⁴ are defined as above.

13. Process for the preparation of substituted glutarimides of the general formula I according to claim 1, in which R⁴ = H and in the group X n and p are defined as above, m represents 1, Z represents O, S or NR⁸ and in the group CR⁸R⁹ at least one of the radicals R⁸ or R⁹ represents hydrogen, **characterised in that** a compound of the general formula VII is added on to a 3-methylene glutarimide of the general formula IX, 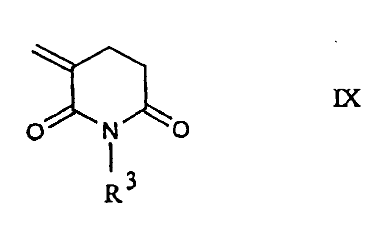 wherein the reaction is preferably carried out in solvents, such as acetonitrile or toluene, with addition of tertiary amines, such as triethylamine or diisopropylethylamine, at temperatures of 80 - 110°C.

14. Process for the preparation of substituted glutarimides of the general formula I according to claim 1, in which R¹ and/or R² represent an amino group, **characterised in that** they are obtained by reduction of compounds of the formula I where R¹ and/or R² = NO₂ the reduction being carried out by catalytically excited hydrogen in acid-containing organic solvents, such as ethyl acetate, preferably using palladium catalysts or with metals, such as tin or iron, in acid solution.

15. Process for the preparation of substituted glutarimides of the general formula I according to claim 1, in which Z in the group X represents SO or SO₂, **characterised in that** they are obtained by stepwise oxidation of the corresponding thioether (Z = S), hydrogen peroxide in acetic acid solution, m-chloroperbenzoic acid, tert-butyl hydroperoxide or oxones being employed as oxidising agents, the latter preferably for the preparation of sulfones (Z = SO₂), or the oxidation to sulfoxides (Z = SO) alternatively being structured asymmetrically by using the Sharpless system or Davis reagent or by means of enzymatic methods.

16. Process for the preparation of substituted glutarimides of the general formula I according to claim 1, in which Z in the group X represents the radical NR⁸, **characterised in that** this radical is converted to the corresponding N-oxide, hydrogen peroxide preferably being used as the oxidising agent.

17. Use of a substituted glutarimide of the formula I according to claim 1 for the preparation of a medicament.

18. Use according to claim 17 for the preparation of a medicament having an immunomodulatory action.

19. Use according to claim 17 for the preparation of a medicament for the treatment of angiopathies.

20. Use according to claim 17 for the preparation of a medicament for the treatment of haematological/ oncological diseases.

## Revendications

1. Glutarimides substitués de formule générale I 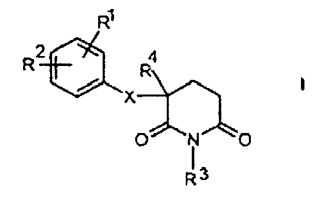 dans laquelle
X est un groupe de formule (CH₂)ₙ-(CR⁸R⁹)ₚ-Z-(CR⁸R⁹)ₘ,
Z représente un atome de soufre ou d'oxygène, le groupe SO ou SO₂ le reste NR⁸ (éventuellement sous forme du N-oxyde) ou un groupe CR³R⁹,
m et p ont la valeur 0 ou 1,
n a la valeur 0, 1, 2 ou 3, m, n et p ne pouvant pas avoir simultanément la valeur 0,
R¹ et R² sont identiques ou différents et représentent le groupe carboxyle, un groupe ester de formule COOR⁵ ou un groupe acyle de formule COR⁵, formules dans chacune desquelles R⁵ représente un groupe alkyle (linéaire ou ramifié) ayant 1 à 6 atomes de carbone (éventuellement substitué avec le reste COOR⁵ et/ou avec un groupe phényle), un groupe cycloalkyle en C₃ à C₇ ou un reste phényle ou benzyle, ou un groupe amide de formule CONR⁶R⁷ dans laquelle R⁶ et R⁷ sont identiques ou différents et représentent l'hydrogène, un groupe alkyle (linéaire ou ramifié) ayant 1 à 6 atomes de carbone (éventuellement substitué avec le reste COOR⁵ et/ou avec un groupe phényle), le reste allyle, le reste phényle ou forment conjointement avec l'atome d'azote le groupe hydrazide, le noyau pyrrolidine, pipéridine, hexaméthylène-imine, morpholine, thiomorpholine, pipérazine ou N-méthylpipérazine, l'hydrogène, le brome, le chlore, le fluor, un groupe mono-, di- ou trifluorométhyle, trityle, hydroxy, hydroxy- méthyle, trifluorométhoxy, nitro, amino (éven- tuellement substitué avec le reste CH(-O) ou COR⁵ ou un groupe alkylsulfonyle) ou diméthylamino, un reste alkyle ou alkoxy (linéaire ou ramifié) ayant 1 à 6 atomes de carbone, un reste amidine de formule NH-CH(=NH) ou NH-C(=NH)R⁵, un reste phényle ou un noyau benzénique condensé (chacun éventuellement substitué avec les atomes ou groupes mentionnés ci-dessus, c'est-à-dire avec l'hydrogène, le brome, le chlore, le fluor, un groupe mono-, di- ou trifluorométhyle, trityle, hydroxy, hydroxyméthyle, trifluorométhoxy, nitro, amino (éventuellement substitué avec le reste CH(=O) ou COR⁵ ou un groupe alkylsulfonyle) ou diméthylamino, un reste alkyle ou alkoxy (linéaire ou ramifié) ayant 1 à 6 atomes de carbone, un reste amidine de formule NH-CH(=NH) ou NH-C(=NH)R⁵ sous réserve que lorsque Z est un groupe CR⁸R⁹, R¹ et R² ne puissent pas représenter simultanément l'hydrogène et que lorsque Z représente S et m est égal à 0, ils ne puissent pas représenter le groupe méthoxy,
R³ représente l'hydrogène, le reste hydroxy ou un groupe de formule CH₂NR⁶R⁷ dans laquelle R⁶ et R⁷ sont tels que définis ci-dessus,
R⁴ représente l'hydrogène, un groupe alkyle en C₁ à C₃, un atome de fluor, le groupe difluoro- ou trifluorométhyle,
R⁸ représente l'hydrogène, un groupe alkyle ayant 1 à 4 atomes de carbone (linéaire ou ramifié), le reste benzyle ou phénéthyle (éventuellement substitué avec les atomes ou les groupes mentionnés ci- dessus, c'est-à-dire avec l'hydrogène, le brome, le chlore, le fluor, un groupe mono-, di- ou trifluorométhyle, trityle, hydroxy, hydroxyméthyle, trifluorométhoxy, nitro, amino (éventuellement substitué avec le reste CH(=O) ou COR⁵ ou un groupe alkylsulfonyle) ou diméthylamino, un reste alkyle ou alkoxy (linéaire ou ramifié) ayant 1 à 6 atomes de carbone, un reste amidine de formule NH-CH(=NH) ou NH-C(=NH)R⁵, et
R⁹ a la même définition que R⁸ et représente le groupe ester de formule COOR⁵ le reste phényle, le groupe hydroxy ou un reste alkoxy (linéaire ou ramifié) ayant 1 à 4 atomes de carbone, un atome de fluor ou de chlore ou le groupe trifluoro- méthyle,
et leurs énantiomères, mélanges d'énantiomères, racémates, diastéréoisomères ou mélanges de diastéréoisomères sous forme de leurs bases ou de leurs sels d'acides physiologiquement acceptables.

2. Glutarimides substitués de formule générale I suivant la revendication 1, **caractérisés en ce que** X représente les groupes CH₂-N et S-CH₂, R¹ représente le groupe carboxyle, un groupe ester de formule COOR⁵ tel que défini ci-dessus, un groupe acyle de formule COR⁵ tel que défini ci-dessus ou un groupe amide de formule CONR⁶R⁷, dans lequel R⁶ et R⁷ sont identiques ou différents et représentent l'hydrogène, un groupe alkyle (linéaire ou ramifié) ayant 1 à 6 atomes de carbone (éventuellement substitué comme défini ci-dessus), le reste phényle, ou forment conjointement avec l'atome d'azote le groupe hydrazide, le noyau pyrrolidine ou morpholidine, R² représente l'hydrogène, le groupe nitro ou amino, R³ représente l'hydrogène et R⁴ représente l'hydrogène, le reste méthyle ou le fluor.

3. Composés suivant la revendication 1:
acide 2-[(3S)-(2,6-dioxo-pipéridine-3-ylamino)méthyl]-benzoïque
acide 2-[(3R)-(2,6-dioxo-pipéridine-3-ylamino)méthyl]-benzoïque 2-[(3S)-(2,6-dioxo-pipéridine-3-ylamino)méthyl]-N,N-diéthyl-benzamide
(3S)-[2-(morpholine-4-carbonyl)benzylamino]pipéridine-2,6-dione
ester éthylique d'acide {2-[(3S)-(2,6-dioxo-pipéridine-3-ylamino)méthyl]-benzoylamino}-acétique
2-[(3S)-(2,6-dioxo-pipéridine-3-ylamino)méthyl]-benzamide
2-[(3S)-(2,6-dioxo-pipéridine-3-ylamino)méthyl]-N-éthyl-benzamide
(3S)-[2-(pyrrolidine-1-carbonyl)benzylamino]pipéridine-2,6-dione
hydrazide d'acide 2-[(3S)-(2,6-dioxo-pipéridine-3-ylamino)-méthyl]-benzoïque
2-[(3S)-(2,6-dioxo-pipéridine-3-ylamino)méthyl]-N-phényl-benzamide
2-[(3R)-(2,6-dioxo-pipéridine-3-ylamino)méthyl]-N-phényl-benzamide
2-[(3R)-(2,6-dioxo-pipéridine-3-ylamino)méthyl]-N,N-diéthyl-benzamide
2-[(3R)-(2,6-dioxo-pipéridine-3-ylamino)méthyl]-benzamide
ester méthylique d'acide 2-[(3S)-(2,6-dioxo-pipéridine-3-ylamino)méthyl]-benzoïque
ester benzylique d'acide 2-[(3S)-(2,6-dioxo-pipéridine-3-ylamino)méthyl]-benzoïque
ester méthylique d'acide 2-(2,6-dioxo-pipéridine-3-yl)-sulfanyl)-benzoïque
ester méthylique d'acide 2-(2,6-dioxo-pipéridine-3-ylméthyl)-sulfanyl-6-nitro)-benzoïque.

4. Médicament contenant comme substance active au moins un composé suivant la revendication 1.

5. Médicament suivant la revendication 4, à action immunomodulatrice et/ou destiné au traitement d'angiopathies et/ou de maladies hématologiques/oncologiques.

6. Procédé de production de glutarimides substitués de formule générale I suivant la revendication 1, dans laquelle R³ représente l'hydrogène ou le reste hydroxy, **caractérisé en ce que** des dérivés d'acide glutarique de formule générale II 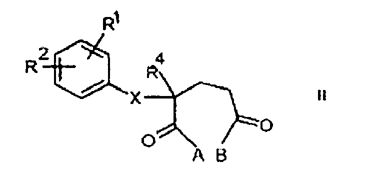 dans laquelle X, R¹, R² et R⁴ ont les mêmes définitions que ci-dessus, soit lorsque A représente OH et B représente NH₂ ou NHOH, soit l'inverse, sont cyclisés en présence de réactifs activateurs, avantageusement de carbonyldiimidazole, ou sont chauffés dans l'acétanhydride lorsque A et B représentent OH et les anhydrides obtenus par cyclisation sont transformés, avec poursuite du chauffage, par réaction avec l'urée ou avec une autre source d'azote, en composés de formule I dans laquelle R³ représente H.

7. Procédé de production de glutarimides substitués de formule générale I suivant la revendication 1, dans laquelle R³ représente l'hydrogène, **caractérisé en ce que** des lactames de formule générale III 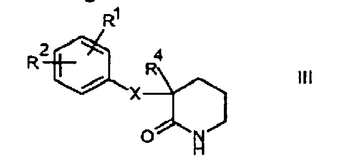 dans laquelle R¹, R², R⁴ et X ont les mêmes définitions que ci-dessus, sont oxydés en imide correspondant, avantageusement avec de l'acide m-chloro-perbenzoïque ou de l'oxyde de ruthénium (IV)/ periodate de sodium.

8. Procédé de production de glutarimides substitués de formule générale I suivant la revendication 1, dans laquelle R³ représente CH₂-NR⁶R⁷, **caractérisé en ce que** des composés de formule I dans laquelle R³ représente H sont amenés à réagir avec le paraformaldéhyde ou une solution aqueuse de formaldéhyde et une amine secondaire de formule HNR⁶R⁷ dans laquelle R⁶ et R⁷ sont tels que définis ci-dessus.

9. Procédé de production de glutarimides substitués de formule générale I suivant la revendication 1, dans laquelle R⁴ représente un groupe alkyle en C₁ à C₃, un atome de fluor, le groupe difluoro- ou trifluorométhyle, **caractérisé en ce que** dans des composés de formule I dans laquelle R⁴ représente H, cet atome d'hydrogène est échangé par des réactions d'alkylation connues contre un groupe alkyle en C₁ à C₃ ou bien le groupe difluoro- ou trifluorométhyle est échangé par des réactions d'halogénation connues contre un atome de fluor.

10. Procédé de production de glutarimides substitués de formule générale I suivant la revendication 1, dans laquelle, pour le groupe X, m est égal à 0 et Z représente NR⁸, R⁸, n et p ayant les mêmes définitions que ci-dessus, **caractérisé en ce que** des α-aminoglutarimides de formule générale IV 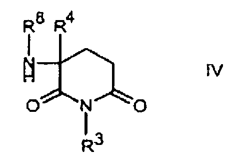 dans laquelle R³, R⁴ et R⁸ ont les mêmes définitions que ci-dessus, sont alkylés avec des composés de formule générale V 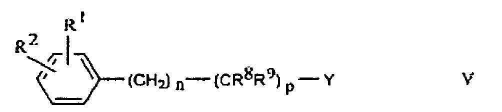 dans laquelle R¹, R², R⁸, R⁹, n et p ont les mêmes définitions que ci-dessus et Y représente un atome de chlore, de brome ou d'iode ou le reste toluène-4-sulfonate.

11. Procédé de production de glutarimides substitués de formule générale I suivant la revendication 1, dans laquelle, pour le groupe X, p est égal à 1 et R⁸ ou R⁹ représente l'hydrogène, **caractérisé en ce qu'**ils sont préparés par amination par voie de réduction à partir de composés de formules générales VI et IV 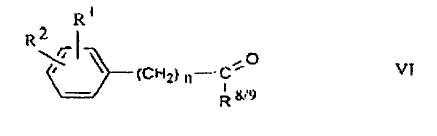 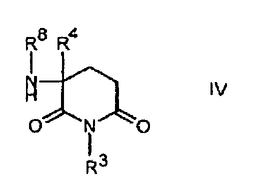 dans lesquelles R¹, R², R⁴, R⁸, R⁹ et n ont les mêmes définitions que ci-dessus et R³ représente l'hydrogène ou le groupe hydroxy, le borohydrure de sodium, le triacétoxy-borohydrure de sodium, le cyanoborohydrure de sodium, le complexe borane-pyridine ou l'hydrogène activé par voie catalytique étant avantageusement utilisé comme agent réducteur.

12. Procédé de production de glutarimides substitués de formule générale I suivant la revendication 1, dans laquelle, pour le groupe X, m est égal à 0 et Z représente O, S ou NR⁹ et R⁸, n et p sont tels que définis ci-dessus, **caractérisé en ce qu'**un composé de formule générale VIII 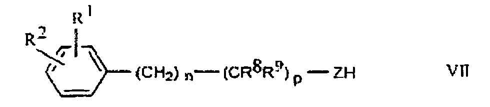 dans laquelle R¹, R² ont les mêmes définitions que ci-dessus, est alkylé avec des α-bromoglutarimides de formule générale VIII 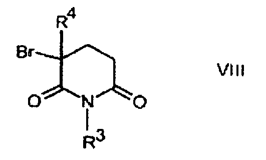 dans laquelle R³ et R⁴ sont tels que définis ci-dessus.

13. Procédé de production de glutarimides substitués de formule générale I suivant la revendication 1, dans laquelle R⁴ représente H et dans le groupe X, n et p sont tels que définis ci-dessus, m est égal à 1, Z représente O, S ou NR⁸ et, dans le groupe CR⁸R⁹, au moins l'un des restes R⁸ et R⁹ représente l'hydrogène, **caractérisé en ce qu'**un composé de formule générale VII est additionné sur un 3-méthylèneglutarimide de formule générale IX, 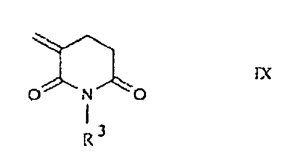 la réaction étant avantageusement conduite dans des solvants tels que l'acétonitrile ou le toluène avec addition d'amines tertiaires telles que la triéthylamine ou la diisopropyl-éthylamine à des températures de 80 à 110°C.

14. Procédé de production de glutarimides substitués de formule générale I suivant la revendication 1, dans laquelle R¹ et/ou R² représentent un groupe amino, **caractérisé en ce qu'**ils sont obtenus par réduction de composés de formule I dans laquelle R¹ et/ou R² représentent NO₂ la réduction étant effectuée par l'hydrogène activé par voie catalytique dans des solvants organiques contenant un acide tels que l'ester éthylique d'acide acétique en utilisant avantageusement des catalyseurs au palladium, mais aussi avec des métaux tels que l'étain ou le fer en solution acide.

15. Procédé de production de glutarimides substitués de formule générale I suivant la revendication 1, dans laquelle Z dans le groupe X représente SO ou SO₂, **caractérisé en ce qu'**ils sont obtenus par oxydation par étapes du thioéther correspondant (Z = S), en utilisant comme agent oxydant le peroxyde d'hydrogène dans une solution d'acide acétique, l'acide m-chloroperbenzoïque, l'hydroperoxyde de tertiobutyle ou l'oxone, cette dernière avantageusement pour la préparation des sulfones (Z = SO₂), ou bien en variante l'oxydation en sulfoxydes (Z = SO) est rendue asymétrique par l'utilisation du système de Sharpless ou du réactif de Davis ou au moyen de méthodes enzymatiques.

16. Procédé de production de glutarimides substitués de formule générale I suivant la revendication 1, dans laquelle Z dans le groupe X représente le reste NR⁸, **caractérisé en ce que** ce reste est converti en N-oxyde correspondant, l'agent oxydant utilisé étant avantageusement le peroxyde d'hydrogène.

17. Utilisation d'un glutarimide substitué de formule I suivant la revendication 1 pour la préparation d'un médicament.

18. Utilisation suivant la revendication 17 pour la préparation d'un médicament à action immunomodulatrice.

19. Utilisation suivant la revendication 17 pour la préparation d'un médicament destiné au traitement d'angiopathies.

20. Utilisation suivant la revendication 17 pour la préparation d'un médicament destiné au traitement de maladies hématologiques/oncologiques.
